Europäisches Patentamt

European Patent Office

Office européen des brevets

(19)

(11) EP 0 981 573 B1

(12) FASCICULE DE BREVET EUROPEEN

(45) Date de publication et mention
de la délivrance du brevet:
**01.09.2004 Bulletin 2004/36**

(21) Numéro de dépôt: **99907719.1**

(22) Date de dépôt: **16.03.1999**

(51) Int Cl.7: **C08J 3/215**, A61L 31/00

(86) Numéro de dépôt international:
**PCT/FR1999/000586**

(87) Numéro de publication internationale:
**WO 1999/047589 (23.09.1999 Gazette 1999/38)**

(54) **EMULSIONS STABLES, LEUR PROCEDE DE PREPARATION ET LEURS APPLICATIONS**

STABILE EMULSIONEN, VERFAHREN ZU DEREN HERSTELLUNG UND IHRE ANWENDUNGEN

STABLE EMULSIONS, PREPARATION METHODS AND APPLICATIONS

(84) Etats contractants désignés:
**BE CH DE DK ES FI FR GB IT LI MC NL SE**

(30) Priorité: **17.03.1998 FR 9803234**

(43) Date de publication de la demande:
**01.03.2000 Bulletin 2000/09**

(73) Titulaire: **HUTCHINSON**
**75008 Paris (FR)**

(72) Inventeurs:
• **CREPEAU, Colette**
**F-60180 Nogent sur Oise (FR)**
• **HOERNER, Pierre**
**F-68180 Horbourg-Wihr (FR)**

• **RIESS, Gérard**
**F-68200 Mulhouse (FR)**

(74) Mandataire: **Vialle-Presles, Marie José et al**
**Cabinet ORES,**
**36,rue de St Pétersbourg**
**75008 Paris (FR)**

(56) Documents cités:
**EP-A- 0 771 837          WO-A-95/17453**

• **E.K.LIN ET AL.: "Semicrystalline Diblock Copolymer Platelets in Dilute Solution" MACROMOLECULES, vol. 29, no. 12, 1996, pages 4432-4441, XP002104880 cité dans la demande**

## Description

La présente invention est relative à des émulsions stables comportant une phase continue formée d'une solution d'élastomère dans un solvant organique et une phase dispersée, sous la forme de gouttelettes de diamètre égal ou supérieur à 10 μm, lesquelles gouttelettes comprennent une substance chimique active dans un solvant non miscible avec la solution d'élastomère et sont aptes à être transformées en films d'élastomère.

La présente invention est également relative au procédé de préparation desdites émulsions ainsi qu'aux films d'élastomère les contenant : lesdites émulsions peuvent conduire, après évaporation du solvant de l'élastomère, à un film élastique renfermant des gouttelettes stables de liquide, de diamètre égal ou supérieur à 10 μm.

Dans la Demande Internationale WO 95/17453 au nom de la Demanderesse, la réalisation d'un matériau solide biphasique contenant une dispersion de gouttelettes liquides d'une substance chimique active, stabilisées au moyen d'un copolymère à blocs ou greffé comportant au moins des séquences polyB compatibles avec lesdites gouttelettes et des séquences polyA, non-miscibles avec ces gouttelettes, dans un élastomère est décrite. Les gouttelettes ont un diamètre généralement inférieur à 10 μm. Dans ces conditions, le copolymère à blocs ou greffé stabilise effectivement l'émulsion, qui est ainsi répartie de manière homogène dans ledit film.

Cette Demande Internationale WO 95/17453 décrit également le procédé de préparation dudit matériau élastomère ; ce procédé comprend essentiellement deux étapes :

**(1)** la préparation d'une émulsion qui comprend :

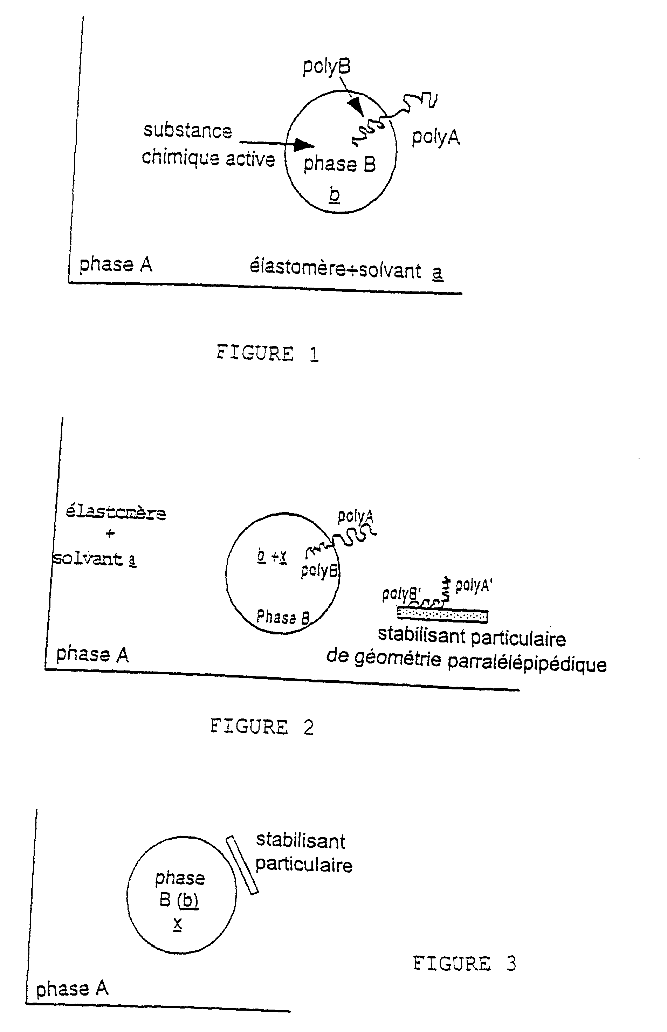

- la préparation d'une phase A par dissolution de l'élastomère dans un solvant organique <u>a</u> (phase A : solution d'élastomère dans le solvant organique <u>a</u>)
- la préparation d'une phase B, par mélange d'une substance chimique active dans un solvant organique <u>b</u> non miscible avec la phase A (phase B : solution ou dispersion de substance active dans un solvant organique <u>b</u> non miscible avec la phase A)
- l'addition à la phase A ou à la phase B, dans des proportions de 0,1 à 50%, de préférence de 0,1 à 25 %, d'un copolymère à blocs ou greffé comportant au moins des séquences polyA, miscibles avec la phase A, et des séquences polyB, miscibles avec la phase B, telles que définies ci-dessus
- la dispersion de la phase B dans la phase A pour l'obtention d'une émulsion dans laquelle la phase A constitue la phase continue et la phase B, la phase dispersée, comme représenté sur la figure 1 et

**(2)** l'évaporation du solvant organique <u>a</u>, pour l'obtention d'un film d'élastomère renfermant sous forme de dispersion stable, des gouttelettes de solvant <u>b</u>, d'un diamètre généralement inférieur à 10 μm, chargé en substance chimique active.

Poursuivant ses recherches, la Demanderesse s'est intéressée plus particulièrement à améliorer la stabilité des émulsions B dans A lors de l'étape **(1)**, notamment celles présentant des gouttelettes de diamètre au moins égal à 10 μm et de préférence comprise entre 10 et 50 μm.

Les différents matériaux élastomères, habituellement utilisés dans le domaine médical ou paramédical (hygiène, notamment) peuvent être modifiés, de manière à être associés à des substances chimiques actives, ayant un effet de protection, lors de l'utilisation de ce matériaux (gants, doigtiers, préservatifs, bandes et pansements divers). En effet, aussi bien dans les cas d'examen ou d'intervention chirurgicale ou en odontologie, que pour la protection à l'encontre d'agents pathogènes, tels que, par exemple bactéries, virus et spores fongiques, une rupture ou même parfois simplement les pores ou une fissure de la membrane d'élastomère, peut entraîner une contamination du porteur dudit matériau par piqûres de seringues, d'aiguilles à suture, de trocart, d'éclats d'os etc..

Or, si les gouttelettes contenant la substance chimique active, qui pourra être libérée en cas de rupture du matériau protecteur en élastomère, ne libèrent pas suffisamment de substance active, l'effet protecteur ne sera pas suffisant.

Par ailleurs, si l'on augmente le diamètre des gouttelettes, de manière à permettre la libération d'une quantité efficace suffisante de substance active, pour neutraliser rapidement l'agent pathogène, qui peut ainsi être accidentellement introduit chez le porteur, le problème de la stabilité de l'émulsion peut se poser de manière cruciale.

Selon la littérature (*P. BECHER, Emulsion : Theory and Practice*, *ACS Monogr., 162*, *1965*), une émulsion peut être définie comme "un système hétérogène constitué d'au moins un liquide immiscible intimement dispersé dans un autre sous la forme de gouttelettes". Ces systèmes contiennent en général un ou plusieurs agents tensioactifs, tels les copolymères blocs ou greffés décrits dans la Demande Internationale WO 95/17453. Dans ce cas, les particules sont soumises à des forces d'attraction de Van der Waals et à des forces de répulsion électrostatiques ou stériques liées à la présence de copolymère, ainsi qu'aux forces de pesanteur, si les densités des phases en présence sont différentes.

L'équilibre entre les forces d'attraction et de répulsion est à l'origine de la stabilisation ou de la déstabilisation des émulsions.

Deux mécanismes de déstabilisation principaux doivent être envisagés pour ces systèmes :

- la floculation, qui se traduit par une agglomération des particules sous la forme de grappes, et
- la coalescence, qui est le résultat de l'amincissement du film liquide séparant les particules et se traduit par une fusion des particules pour former une particule unitaire de taille plus élevée.

Les copolymères à blocs ou greffés, tels que décrits dans la Demande Internationale WO 95/17453 sont particulièrement adaptés à la stabilisation des émulsions comprenant des gouttelettes de diamètre inférieur ou égal à 10 µm, en réduisant les phénomènes de coalescence ou/et de floculation.

La Demanderesse a maintenant trouvé que pour stabiliser des gouttelettes d'une phase B (contenant une substance chimique active $\underline{x}$ et un solvant $\underline{b}$) d'un diamètre $\geq$ 10 µm, dispersées dans une phase organique A (élastomère + solvant $\underline{a}$), lesdits copolymères à blocs ou greffés doivent être utilisés en mélange avec d'autres composés de type *stabilisant particulaire*, pour effectivement obtenir une stabilisation de l'émulsion.

La Demanderesse a également trouvé que, lorsque l'émulsion comprend certaines substances chimiques $\underline{x}$ possédant des propriétés tensioactives, il est possible de réduire la teneur en copolymère à blocs ou greffé, voire de s'en affranchir. Dans un tel cas, ladite substance chimique, incluse dans la phase B et associée au stabilisant particulaire, permet également d'obtenir une émulsion stable.

La présente invention a pour objet une émulsion stable d'au moins une substance chimique $\underline{x}$ dans une solution d'élastomère, apte à être utilisée pour la préparation d'un film d'élastomère, comprenant (1) une phase A comprenant un élastomère dissous dans un solvant organique $\underline{a}$ apolaire ou peu polaire, dans laquelle est dispersée (2) une phase B comprenant au moins ladite substance chimique $\underline{x}$, en solution ou dispersée dans un solvant $\underline{b}$ polaire, non miscible avec la phase A et (3) au moins un agent dispersant sélectionné dans le groupe constitué par les copolymères à blocs ou greffés, laquelle émulsion est caractérisée :

- en ce que les gouttelettes de phase dispersée B ont un diamètre $\geq$ 10 µm,
- en ce que ladite émulsion comprend, pour la stabilisation de ladite phase dispersée B, outre au moins un copolymère à blocs ou greffé (également dénommé *copolymère dispersant*) comportant des séquences poly A, compatibles avec la phase A et des séquences poly B compatibles avec la phase B, au moins un *stabilisant particulaire* sélectionné dans le groupe constitué par des composés solides organiques de dimension comprise entre 30 nm et 10 µm ou des composés solides minéraux de dimension comprise entre 5 nm et 10 µm, de géométrie quelconque (sphère, parallélépipède, solide finement divisé...), dont l'état de surface est organophile,
- en ce que la fraction massique $\phi_B$ de phase dispersée (phase B) dans l'émulsion, exprimée par :

$$\phi_B = \frac{m_B}{m_B + m_A + m_{\text{élastomère}}}$$

est comprise entre 0,01 et 0,2,

avec $m_B$=masse de phase B (solvant $\underline{b}$ + substance chimique active $\underline{x}$)

$m_A$= masse de solvant $\underline{a}$

$m_{\text{élastomère}}$= masse de l'élastomère dissous dans $\underline{a}$ ;

- en ce que la fraction massique du copolymère à blocs ou greffé, (*copolymère dispersant, CD*), $\phi_{CD}$, exprimée par rapport à la phase dispersée B, à savoir :

$$\phi_{CD} = \frac{m_{CD}}{m_{CD} + m_B}$$

est comprise entre 0,001 et 0,3 de préférence entre 0,01 et 0,2,

avec $m_B$=masse de phase B (solvant $\underline{b}$ + substance chimique active $\underline{x}$)

$m_{CD}$=masse de copolymère dispersant ; et

- en ce que la fraction massique de *stabilisant particulaire* (SP), $\phi_{SP}$, formé *in situ* ou ajouté en tant qu'adjuvant, exprimée par rapport à la phase dispersée B, à savoir :

$$\phi_{SP} = \frac{m_{SP}}{m_{SP} + m_B}$$

est comprise entre 0,001 et 0,5,

avec $m_{SP}$= masse de *stabilisant particulaire*

$m_B$= masse de phase B (solvant b + substance chimique active x).

Pour des raisons de commodité, les proportions des différents constituants de l'émulsion sont exprimées en fractions massiques et non en fractions volumiques.

De façon préférée, ladite fraction massique du copolymère à blocs ou greffé, $\phi_{CD}$, est comprise entre 0,001 et 0,2, de préférence entre 0,01 et 0,1.

On entend par *stabilisant particulaire* dont l'état de surface est organophile, un composé solide organique, en particulier de type polymère, ou minéral, finement divisé et dispersable dans la phase continue (phase A) ; l'état de surface peut être modifié par un agent de traitement de surface, pour rendre ledit *stabilisant particulaire* organophile.

Dans de telles émulsions, le copolymère à blocs ou greffé désigné par *copolymère dispersant* sert essentiellement à faciliter la dispersion d'un liquide dans l'autre au moment de la préparation de l'émulsion, par abaissement de la tension interfaciale entre la phase A et B, et seulement partiellement à la stabilisation des gouttelettes puisque cette dernière est apportée pour l'essentiel par le *stabilisant particulaire*.

De manière surprenante, la combinaison d'un *copolymère dispersant* et d'un *stabilisant particulaire* permet de stabiliser une émulsion comportant des gouttelettes de diamètre ≥ 10 µm. En effet, dans un tel cas, le *stabilisant particulaire* à lui seul n'a pas d'effet dispersant et ne permet pas de réaliser l'émulsion ; de même, le *copolymère dispersant* seul ne stabilise pas suffisamment l'émulsion.

Un schéma synoptique représentant une gouttelette d'émulsion stabilisée par un mélange de *copolymère dispersant* et d'un *stabilisant particulaire* est représenté à la figure 2.

Conformément à l'invention, lorsque le *stabilisant particulaire* est de nature organique, il est essentiellement de type polymère, et est sélectionné dans le groupe constitué par des copolymères à blocs ou greffé organophiles, identiques ou différents de ceux utilisés comme *copolymères dispersants,* aptes à former des structures d'un diamètre supérieur à 30 nm et des dérivés de la cellulose comme la microcellulose, l'amidon, ou certains polymères finement divisés ; dans ce cas, ledit *stabilisant particulaire* est :

- soit naturellement organophile (copolymères à blocs ou greffés), du fait de la présence d'une chevelure de séquences polyA, à sa surface ; il peut être alors :

  * formé *in situ* dans le solvant a de la phase A ; dans ce cas, il est de préférence identique au *copolymère dispersant* et il est sélectionné parmi les copolymères à blocs capables de former, en plus des micelles "classiques", d'autres structures figées, de taille supérieure à 30 nm, comme par exemple des structures de type "carreaux" (détaillées dans l'exemple 1) ou tout autre agrégat de copolymère de géométrie moins bien définie. Ce sont toutes ces structures associées et figées qui peuvent se comporter tel un *stabilisant particulaire*. De préférence, un tel *stabilisant particulaire* est sélectionné dans le groupe constitué par les copolymères contenant une séquence polyB cristallisable, comme par exemple polyoxyéthylène, poly(éthylène), polyamide ou polyester tel que poly(caprolactone), sachant que la séquence polyA doit être soluble dans la phase A de l'émulsion ou
  * ajouté sous la forme de structures associées et figées de taille supérieure à 30 nm, formées par un copolymère à blocs, et préalablement isolées des autres structures de faible taille telles que les micelles. Dans ce cas, le *stabilisant particulaire* peut être éventuellement de nature chimique différente du *copolymère dispersant,* mais est sélectionné parmi les *stabilisants particulaires* tels que définis ci-dessus.

- soit rendu organophile et ajouté en tant qu'adjuvant avant, pendant ou après formation de l'émulsion ; il est sélectionné parmi les dérivés de la cellulose comme la microcellulose, l'amidon, ou certains polymères finement divisés ; il est rendu organophile par traitement de sa surface par un agent de traitement de surface apte, à le rendre organophile et donc dispersable dans la phase A de l'émulsion, et à lui conférer des propriétés de stabilisation par voie stérique ; la fraction massique de l'agent de traitement de surface, utilisé pour rendre la surface des particules organophile, soit $\phi_{ATS,}$ exprimée par rapport au *stabilisant particulaire*, à savoir :

$$\phi_{ATS} = \frac{m_{ATS}}{m_{SP} + m_{ATS}}$$

est comprise entre 0,001 et 0,5,

avec $m_{ATS}$=masse de l'agent de traitement de surface

$m_{SP}$= masse de *stabilisant particulaire*.

De façon préférée, ladite fraction massique de l'agent de traitement de surface, $\phi_{ATS,}$ est comprise entre 0,001 et 0,1.

Egalement conformément à l'invention, lorsque le *stabilisant particulaire* est de type minéral, ce dernier est choisi dans le groupe qui comprend les argiles, les silices, le talc, le kaolin ou des dérivés de ces produits et est traité en surface par un agent de traitement de surface, apte à le rendre organophile et donc dispersable dans la phase continue (phase A) de l'émulsion, et à lui conférer des propriétés de stabilisation par voie stérique et est ajouté, tel un adjuvant, à la phase A contenant la solution d'élastomère dans le solvant a et le *copolymère dispersant* ; la fraction massique d'agent de traitement de surface par rapport au *stabilisant particulaire* à l'émulsion est comprise entre 0,001 et 0,5, comme précisé ci-dessus.

De façon préférée, ladite fraction massique de l'agent de traitement de surface, $\phi_{ATS,}$ est comprise entre 0,001 et 0,1.

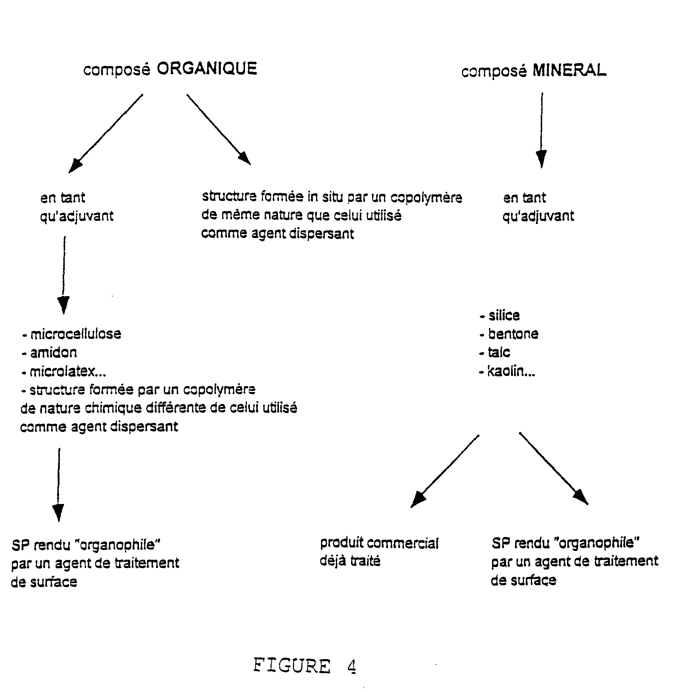

En variante, le *stabilisant particulaire* minéral peut être choisi parmi les produits "organophiles" disponibles commercialement, comme par exemple certaines silices ou argiles organophiles (Bentone 38, Bentone SD-1 RHEOX INC.,...), et de ce fait ne nécessiter aucun agent de traitement de surface supplémentaire. Ces différentes possibilités sont résumées dans la figure 4.

Selon un mode de réalisation avantageux de ladite émulsion, elle comprend plusieurs *stabilisants particulaires*, comme par exemple la combinaison d'un *stabilisant particulaire* formé *in situ* lors de la mise en solution du copolymère dispersant avec un second *stabilisant particulaire* sous forme d'adjuvant.

Selon un autre mode de réalisation avantageux de ladite émulsion, le copolymère à blocs dispersant est sélectionné parmi les copolymères di-bloc, de type polyA-bloc-polyB, les copolymères tribloc de type polyB-bloc-polyA-bloc-polyB (BAB), de type polyA-bloc-polyB-bloc-polyA (ABA), de type polyA-bloc-polyB-bloc-polyC (ABC) ou polyA-bloc-polyC-bloc-polyB (ACB), ou plus généralement parmi les composés multibloc contenant des séquences polyA, polyB et polyC et le copolymère du type greffé dispersant est sélectionné parmi les composés de type polyA-greffé-polyB, polyB-greffé-polyA, de type polyA-greffé-polyB et polyC ou de type polyC-greffé-polyA et polyB.

Les proportions de séquence polyA, exprimées en masse par rapport à la somme des séquences polyA+polyB, sont comprises entre 10 et 90 %, et les proportions de séquences polyB sont comprises entre 90 et 10 %, et les proportions massiques de séquences polyC sont comprises entre 0 et 50 % par rapport à l'ensemble des séquences.

Les masses molaires des séquences poly A, poly B et poly C sont comprises entre 1 000 et 500 000 Daltons.

Conformément à l'invention, les séquences poly A, compatibles avec le solvant apolaire ou peu polaire a, sont sélectionnées dans le groupe constitué par les polydiènes, les polyoléfines, les polyéthers ou les silicones, tels le polyisoprène, le polybutadiène, le polyisobutène, le polybutadiène hydrogéné ou le polyisoprène hydrogéné, le poly(4-tertiobutylstyrène), le polyoxypropylène, le polyoxybutylène, le polydiméthylsiloxane, le poly(méthacrylate de 2-ethyle hexyl), le poly(méthacrylate de lauryle) miscibles avec une solution d'élastomère dans un solvant a, les séquences polyB, compatibles avec le solvant b, sont sélectionnées dans le groupe constitué par le polyoxyéthylène, la polyvinylpyrrolidone, les polyacides acryliques, le poly(alcool vinylique) et le poly(vinylpyridine) quaternisé.

Les séquences polyC peuvent être choisies dans le groupe qui comprend les polymères pouvant être soit compatibles avec le solvant a ou le solvant b, soit non compatibles avec les solvants a et b. La séquence polyC est choisie et ce de manière non limitative dans le groupe formé par les polymères acryliques ou vinyliques tels le poly(méthacrylate de méthyle) ou le polystyrène.

Selon un autre mode de réalisation avantageux de ladite émulsion, l'agent de traitement de surface, apte à rendre le *stabilisant particulaire* organophile, est sélectionné dans le groupe constitué par des copolymères à blocs ou greffé contenant au moins une séquence polyA', soluble dans le solvant a, et une séquence polyB' capable de s'adsorber ou de s'ancrer à la surface du *stabilisant particulaire*, lesdites séquences poly A' et poly B' étant identiques ou différentes des séquences poly A et poly B et des oligomères fonctionnalisés de type polyA'-F, dans lesquels F est une fonction chimique capable de s'adsorber sur le *stabilisant particulaire*.

Conformément à l'invention, les séquences polyA' sont choisies parmi les polymères solubles dans le solvant apolaire ou peu polaire a, comme par exemple dans le groupe qui comprend les polydiènes, les polyoléfines, les polyéthers ou les silicones, tels le polyisoprène, le polybutadiène, le polyisobutène, le polybutadiène hydrogéné ou le polyisoprène hydrogéné, le poly(4-tertiobutylstyrène), le polyoxypropylène, le polyoxybutylène, le polydiméthylsiloxane, le poly(méthacrylate de 2-ethyle hexyl), le poly(méthacrylate de lauryle) miscibles avec une solution d'élastomère dans un solvant a, et les séquences poly B' sont choisies parmi les polymères capables de s'adsorber à la surface du *stabilisant particulaire*, et est choisi dans le groupe qui comprend le polyoxyéthylène, la polyvinylpyrrolidone, les polyacides acryliques, le poly(alcool vinylique) et le poly(vinylpyridine) quatemisé ou non.

Selon un autre mode de réalisation avantageux de l'émulsion, les proportions de séquence polyA', exprimées en masse par rapport à la somme des séquences polyA'+polyB', sont comprises entre 10 et 90 %, de préférence entre 20 et 80 % et les proportions de séquences polyB' sont comprises entre 90 et 10 %.

Selon encore un autre mode de réalisation avantageux de ladite émulsion, les masses molaires des séquences polyA' et polyB' sont comprises entre 150 et 200 000 Daltons.

Selon un autre mode de réalisation avantageux de ladite émulsion, F est sélectionné dans le groupe constitué par les fonctions acides, amines ou alcools et les groupements aptes à réagir chimiquement avec la surface du *stabilisant particulaire*, comme par exemple des groupements époxy, isocyanate ou aziridine.

De telles émulsions d'une substance chimique active x dans une solution d'élastomère, comprennent des gouttelettes qui présentent un diamètre égal ou supérieur à 10 μm et une très bonne stabilité conférée conjointement par ledit copolymère à blocs ou greffé et par ledit *stabilisant particulaire* rendu organophile par un agent de traitement de surface tel que défini ci-dessus.

La stabilité desdites émulsions peut être évaluée par la détection des phénomènes de coalescence et de floculation, ainsi que par le suivi du diamètre des gouttelettes au cours du temps.

L'élastomère, le solvant a, la substance chimique active x et le solvant b sont notamment ceux décrits dans la Demande Internationale WO 95/17453.

Par exemple :

- l'élastomère est sélectionné, et ce de manière non limitative, dans le groupe constitué par le polybutadiène, le polyisoprène, le polychloroprène, les copolymères SBR (S*tyrene Butadiene Rubber*), NBR (*Nitrile Butadiene Rubber*), SBS (*Styrene Butadiene Styrene*), SIS (*Styrene Isoprene Styrene*), SEBS (*Styrene Ethylene-co-Butylene Styrene*) seul ou en mélange avec un ou plusieurs plastifiants ou flexibilisants.
- le solvant a, apolaire ou peu polaire, est choisi notamment parmi les hydrocarbures aliphatiques, aromatiques et alicycliques, par exemple le méthylcyclohexane, le toluène, l'heptane, ou un mélange de ceux-ci.
- la substance chimique active x est sélectionnée parmi les composés capables de provoquer une dénaturation quasi-instantanée des protéines par simple contact, soit par réaction chimique, soit par effet physico-chimique tel qu'une modification de la tension de surface. Cette famille de composés comprend entre autres les biocides, tels que les ammonium quaternaires, de préférence du chlorure de diméthyldidécylammonium, des biguanides, le phtalaldéhyde, des dérivés phénoliques ou benzyliques, le formol, des tensioactifs non-ioniques comportant au moins une séquence polyoxyéthylene, l'hexamidine, des composés iodés de polyvinylpyrrolidone, des tensioactifs non-ioniques à activité virucide, les bichromates et hypochlorites de sodium et de potassium, utilisés seuls ou en mélange.
- le solvant b est non miscible avec le solvant a tel que défini ci-dessus. b est sélectionné, par exemple, parmi les polyols, et de préférence la glycérine, l'éthylène glycol et les polyéthylènes glycols liquides à température ambiante et de masse molaire comprise entre 62 (éthylène glycol) et 750 Daltons (PEG 750) mais peut être tout autre composé non miscible avec le solvant a comme par exemple l'eau, la diméthylsulfoxyde, la formamide ou l'éthanolamine ou les mélanges de tels solvants.

De manière préférée, la fraction massique de substance chimique active x, soit $\phi_x$, dans l'émulsion décrite ci-dessus est exprimée par

$$\phi_x = \frac{m_x}{m_x + m_b} = \frac{m_x}{m_B}$$

et est comprise entre 0,01 et 0,7, de préférence entre 0,1 et 0,4,

avec $m_x$ = masse de substance chimique active x

$m_b$= masse de solvant $\underline{b}$

$m_B$= masse de phase dispersée B.

En variante, si la substance chimique active $\underline{x}$ a des propriétés tensioactives, c'est-à-dire si elle permet d'abaisser la tension interfaciale entre la phase A et la phase B, ce qui est notamment le cas des ammoniums quaternaires, des tensioactifs non-ioniques comportant au moins une séquence polyoxyéthylène et des tensioactifs non-ioniques à activité virucide, cette substance chimique active $\underline{x}$ peut jouer le rôle d'agent dispersant. Il en résulte que, dans ces conditions, il est possible de réduire la teneur en copolymère à blocs ou greffé dans l'émulsion décrite ci-dessus, voire de s'en affranchir, simplifiant ainsi la formulation de l'émulsion selon l'invention tout en assurant une stabilisation convenable des gouttelettes de diamètre $\geq$ 10 µm.

La présente invention a donc également pour objet une émulsion stable d'au moins une substance chimique $\underline{x}$ dans une solution d'élastomère, apte à être utilisée pour la préparation d'un film d'élastomère, comprenant (1) une phase A comprenant un élastomère dissous dans un solvant organique $\underline{a}$ apolaire ou peu polaire, dans laquelle est dispersée (2) une phase B comprenant au moins ladite substance chimique $\underline{x}$, en solution ou dispersée dans un solvant $\underline{b}$ polaire, non miscible avec la phase A , laquelle émulsion est caractérisée :

- en ce que les gouttelettes de phase dispersée B ont un diamètre $\geq$ 10 µm,
- en ce que ladite émulsion comprend, pour la stabilisation de ladite phase dispersée B, **(1)** au moins une substance chimique $\underline{x}$, cette dernière ayant des propriétés tensioactives et jouant le rôle d'agent dispersant, ladite substance chimique $\underline{x}$ étant sélectionnée dans le groupe constitué par les ammoniums quaternaires, les tensioactifs non-ioniques comportant au moins une séquence polyoxyéthylène et les tensioactifs non-ioniques à activité virucide, et **(2)** au moins un *stabilisant particulaire* tel que défini précédemment, de préférence formé à partir d'un copolymère à blocs ou greffé,
- en ce que la fraction massique $\phi_B$ de phase dispersée (phase B) dans l'émulsion, exprimée par

$$\phi_B = \frac{m_B}{m_B + m_A + m_{élastomère}}$$

est comprise entre 0,01 et 0,2,

avec $m_B$=masse de phase B (solvant $\underline{b}$ + substance chimique active $\underline{x}$)

$m_A$= masse de solvant $\underline{a}$

$m_{élastomère}$= masse de l'élastomère dissous dans $\underline{a}$ ;

- en ce que la fraction massique de la substance chimique active $\underline{x}$, soit $\phi_x$ , exprimée par

$$\phi_x = \frac{m_x}{m_x + m_b} = \frac{m_x}{m_B}$$

est comprise entre 0,01 et 0,7, de préférence entre 0,1 et 0,4,

avec $m_x$= masse de substance chimique active $\underline{x}$

$m_b$= masse de solvant $\underline{b}$

$m_B$= masse de phase dispersée B,

- en ce que la fraction massique de *stabilisant particulaire* (SP), $\phi_{SP}$, formé *in situ* ou ajouté en tant qu'adjuvant, exprimée par rapport à la phase dispersée B :

$$\phi_{SP} = \frac{m_{SP}}{m_{SP} + m_B}$$

est comprise entre 0,001 et 0,5,

avec $m_{SP}$= masse de *stabilisant particulaire*

$m_B$= masse de phase B.

De façon avantageuse, ledit élastomère, ledit solvant a, ledit solvant b et ledit stabilisant particulaire sont tels que définis précédemment dans le cadre des émulsions comprenant un agent dispersant sélectionné dans le groupe constitué par les copolymères à blocs ou greffés.

De manière surprenante, la combinaison de la substance chimique active x possédant des propriétés tensioactives et du ou des stabilisants particulaires, de préférence lorsque ceux-ci sont des copolymères à blocs ou greffés tels que définis précédemment, permet de stabiliser une émulsion comportant des gouttelettes de diamètre $\geq 10\ \mu m$.

La présente invention a également pour objet un procédé de préparation de ladite émulsion stable :

I. Dans le cas où le *stabilisant particulaire* est ajouté avant la formation de l'émulsion, cette dernière est réalisée comme suit :

- la préparation d'une phase A par dissolution de l'élastomère dans un solvant organique a (phase A : solution d'élastomère dans le solvant organique a),
- la préparation d'une phase B, par mélange d'une substance chimique active x dans un solvant organique b non miscible avec la phase A (phase B : solution ou dispersion de substance active dans un solvant organique b non miscible avec la phase A),
- l'addition à la phase A ou à la phase B, dans les proportions telles que définies ci-dessus d'un copolymère à blocs ou greffé servant essentiellement de *copolymère dispersant* ; toutefois, si ce copolymère est capable de former *in situ,* dans la phase A, en plus d'une organisation classique de type micellaire, d'autres structures associées et figées, d'un diamètre supérieur à 30 nm, pouvant jouer le rôle de *stabilisant particulaire*, ce dernier n'a plus besoin d'être ajouté dans une étape ultérieure ; lorsque ledit copolymère comprend une séquence polyB cristallisable, il est effectivement capable de former *in situ,* dans la phase A, en plus d'une organisation classique de type micellaire, d'autres structures associées et figées pouvant jouer le rôle de *stabilisant particulaire*. Dans le cas contraire, l'addition à la phase A de la fraction massique du *stabilisant particulaire*, telle que définie ci-dessus, s'effectue dans un second temps, sous forme d'adjuvant. Ce composé solide, organique ou minéral est organophile et donc, si nécessaire, traité préalablement par un agent de traitement de surface, comme précisé ci-dessus, et
- la dispersion de la phase B dans la phase A, pour l'obtention d'une émulsion dans laquelle la phase A constitue la phase continue et la phase B, la phase dispersée.

Dans le cas où la substance chimique active x joue le rôle d'agent dispersant, le procédé de préparation de l'émulsion s'effectue comme suit, dans le cas où le *stabilisant particulaire* est ajouté avant la formation de l'émulsion :

- la préparation d'une phase A par dissolution de l'élastomère dans un solvant organique a (phase A : solution d'élastomère dans le solvant organique a),
- la préparation d'une phase B, par mélange de la substance chimique active x à caractère tensioactif dans un solvant organique b non miscible avec la phase A (phase B : solution ou dispersion de substance active dans un solvant organique b non miscible avec la phase A),
- l'addition à la phase A ou à la phase B, dans les proportions telles que définies ci-dessus, d'un *stabilisant particulaire* organophile ou rendu organophile, de préférence formé à partir d'un copolymère à blocs ou greffé,
- la dispersion de la phase B dans la phase A pour l'obtention d'une émulsion dans laquelle la phase A constitue la phase continue et la phase B, la phase dispersée.

II. Dans le cas où le *stabilisant particulaire* est ajouté après la formation de l'émulsion, cette dernière est réalisée comme suit :

- la préparation d'une phase A par dissolution de l'élastomère dans un solvant organique a (phase A : solution d'élastomère dans le solvant organique a),
- la préparation d'une phase B, par mélange d'une substance chimique active x dans un solvant organique b non miscible avec la phase A (phase B : solution ou dispersion de substance active dans un solvant organique b non miscible avec la phase A),

EP 0 981 573 B1

- l'addition à la phase A ou à la phase B, dans des proportions telles que définies ci-dessus, d'un copolymère à blocs ou greffé servant essentiellement de *copolymère dispersant*,
- la dispersion de la phase B dans la phase A pour l'obtention d'une émulsion dans laquelle la phase A constitue la phase continue et la phase B, la phase dispersée,
- l'ajout, sous agitation, du *stabilisant particulaire* organophile tel que défini précédemment.

Dans le cas où la substance chimique active x joue le rôle d'agent dispersant, le procédé de préparation de l'émulsion s'effectue comme suit, dans le cas où le *stabilisant particulaire* est ajouté pendant ou après la formation de l'émulsion :

- la préparation d'une phase A par dissolution de l'élastomère dans un solvant organique a (phase A : solution d'élastomère dans le solvant organique a),
- la préparation d'une phase B, par mélange de la substance chimique active x à caractère tensioactif dans un solvant organique b non miscible avec la phase A (phase B : solution ou dispersion de substance active dans un solvant organique b non miscible avec la phase A),
- la dispersion de la phase B dans la phase A pour l'obtention d'une émulsion dans laquelle la phase A constitue la phase continue et la phase B, la phase dispersée, et l'ajout simultané ou non, sous agitation, du *stabilisant particulaire* organophile ou rendu organophile, de préférence formé à partir d'un copolymère à blocs ou greffé.

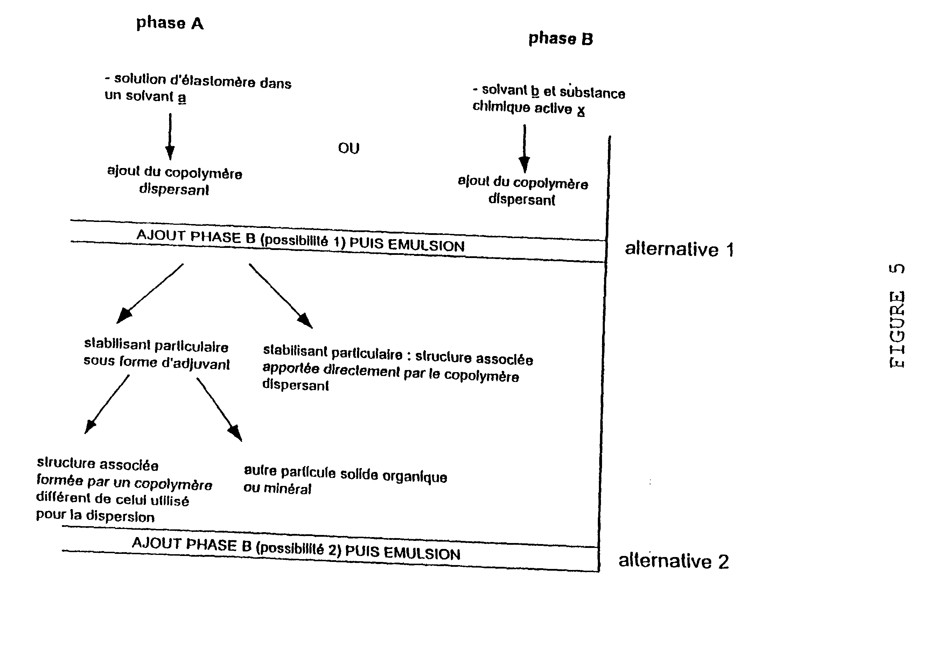

En variante, le *stabilisant particulaire* est ajouté pendant la formation de l'émulsion, c'est-à-dire au moment où la phase B est dispersée dans la phase A.
En variante, le *stabilisant particulaire* peut être rendu organophile directement par le copolymère utilisé pour la dispersion de l'émulsion.
Ces différentes alternatives sont résumées à la figure 5.
La présente invention a également pour objet un film d'élastomère,
caractérisé en ce qu'il est obtenu par évaporation du solvant a, à partir d'une émulsion telle que définie ci-dessus.
Un tel film d'élastomère, qui peut avantageusement servir de support protecteur, comprend des gouttelettes stables d'un solvant b non miscible avec l'élastomère, chargées en au moins une substance active x, lesquelles gouttelettes ont un diamètre $\geq 10$ µm, sont uniformément réparties dans l'ensemble du matériau élastomérique.
Conformément à l'invention, l'association d'au moins un copolymère à blocs ou greffé et d'au moins un stabilisant particulaire permet d'obtenir la stabilité de l'émulsion aussi bien en présence du solvant a qu'au cours de l'évaporation dudit solvant a, c'est-à-dire lors de la formation du film, qui, lui, est stable et présente des caractéristiques mécaniques optimales.
La présente invention a également pour objet un procédé de préparation dudit film d'élastomère, caractérisé en ce qu'il comprend :

(a) la préparation d'une émulsion telle que définie ci-dessus et
(b) l'évaporation du solvant organique a, pour l'obtention d'un film d'élastomère renfermant sous forme de dispersion stable, des gouttelettes de solvant b, chargées en substance chimique active x, d'un diamètre $\geq 10$ µm.

La présente invention a également pour objet les différentes applications du film d'élastomère selon l'invention, plus particulièrement dans le domaine médical et paramédical : gants, doigtiers, préservatifs ou pansements comportant un film d'élastomère conforme à l'invention.
Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 est une représentation schématique de l'émulsion dans l'étape (1) telle que décrite dans la Demande Internationale PCT WO 95/17453;
- la figure 2 est une représentation schématique de l'émulsion selon l'invention, le *stabilisant particulaire* étant organique ou minéral et de géométrie parralélépipédique, le copolymère dispersant étant un copolymère dibloc polyA-polyB ;
- la figure 3 est une représentation schématique de l'émulsion selon l'invention, dans laquelle la substance chimique x joue le rôle d'agent dispersant ;
- la figure 4 représente les différentes options de *stabilisant particulaire* (SP) : solide organique ou minéral finement divisé ;
- la figure 5 illustre un diagramme des différentes alternatives de préparation des émulsions selon l'invention ;
- la figure 6 représente une structure schématique en " carreaux " d'un copolymère contenant une séquence polyB cristalline insoluble dans le solvant <u>a</u>, et une séquence polyA soluble dans <u>a</u> ;
- la figure 7 représente la stabilisation d'une émulsion par un *stabilisant particulaire* organique (structure associée de type carreau formée par un copolymère PBut-POE), et par un copolymère dispersant de nature chimique identique à celle du *stabilisant particulaire*.

Il doit être bien entendu, toutefois, que ces exemples sont donnés uniquement à titre d'illustration de l'objet de l'invention, dont ils ne constituent en aucune manière une limitation.

## Exemple 1 : Evaluation de la stabilité et du diamètre des émulsions obtenues selon l'invention.

La stabilité et le diamètre des émulsions sont évalués dans les conditions précisées ci-après et ce, compte tenu des paramètres suivants.
Une émulsion telle que définie dans la présente invention est soumise à plusieurs forces que sont :

- les forces d'attraction entre les gouttelettes, qui peuvent engendrer de la floculation et/ou de la coalescence ;
- les forces de répulsion entre les gouttelettes, par la présence du copolymère adsorbé à leur surface et par la présence du *stabilisant particulaire* organophile qui réduit les collisions entre les gouttelettes.

C'est la présence des forces de répulsion qui permet de ralentir voire d'éviter les phénomènes de coalescence et/ou de floculation.
Par définition, une émulsion sera considérée comme stable lorsqu'elle ne coalesce ni ne flocule.
La stabilité des émulsions est évaluée au moyen de deux techniques complémentaires :

- par suivi direct des phénomènes de floculation et/ou de coalescence à l'aide d'un dispositif fonctionnant par diffusion et rétrodiffusion de la lumière (appareil commercialisé par la société FORMULACTION sous le nom TURBISCAN MA 1000). L'apparition de phénomènes de déstabilisation est détectée par une modification du signal transmis ou rétrodiffusé par l'émulsion.
- par suivi du diamètre moyen au cours du temps : si l'émulsion est parfaitement stable, le diamètre est constant au cours du temps. inversement, il augmente sous l'effet de la floculation ou de la coalescence des gouttelettes.

Pour ce faire, le diamètre de l'émulsion est mesuré par la technique de photosédimentation sur une partie aliquote de l'émulsion, sur un appareil du type SHIMADZU SA-CP3.
Cette technique permet d'avoir accès à la distribution en taille de l'émulsion. Dans cette étude, le diamètre de l'émulsion sera exprimé par le diamètre modal des particules, qui correspond au maximum de la courbe de distribution.

## Exemple 2 : Émulsion préparée en présence d'un copolymère dibloc polybutadiène-polyoxyéthylène, noté PBut-POE (copolymère dispersant) et d'un *stabilisant particulaire* organique formé in situ à partir de ce copolymère dibloc : Comparaison avec les caractéristiques de l'émulsion obtenue en absence de *stabilisant particulaire*.

Dans cet exemple, le *stabilisant particulaire* correspond à une structure figée formée in situ par une fraction du *copolymère dispersant* en solution dans le solvant <u>a</u>.
La préparation d'une émulsion stable et de granulométrie élevée conformément à l'invention s'effectue comme suit :

- Préparation de la phase continue (phase A) :

On dissout, sous agitation, du SEBS (copolymère tribloc poly-styrène-poly(éthylène/co/butylène)-polystyrène) de synthèse de masse molaire de 85 000 Daltons et contenant 29 % en masse de polystyrène, commercialisé par la société SHELL sous le nom KRATON G 1652 de manière à obtenir une solution à 20 % en poids en SEBS.

- Préparation de la phase dispersée (phase B) :

On dissout, sous agitation, du Bardac (chlorure de diméthyldidécylammonium) dans du polyethylène glycol de masse molaire 400 Daltons (PEG 400) de manière à obtenir une solution à 30 % en masse en Bardac.

- Préparation de la solution comportant le *copolymère dispersant* et le *stabilisant particulaire* :

Un copolymère dibloc polybutadiène-polyoxyéthylène, noté PBut-POE, a été synthétisé par polymérisation anionique (M. Gervais et al., *Makromol. Chem.*, 1977, **178,** 1577-1593). Sa teneur massique en POE (déterminée par RMN $^1$H) est de 60 %, sa masse molaire totale de 10 000 Daltons.

La mise en solution du copolymère s'effectue selon un protocole couramment décrit dans la littérature (E. K. Lin et al., *Macromolecules,* 1996, **29,** 4432-4441). Dans cet exemple, 10 grammes de ce copolymère sont mis en solution dans 90 grammes de méthylcyciohexane.

Après solubilisation du copolymère, deux types de structures sont formées :

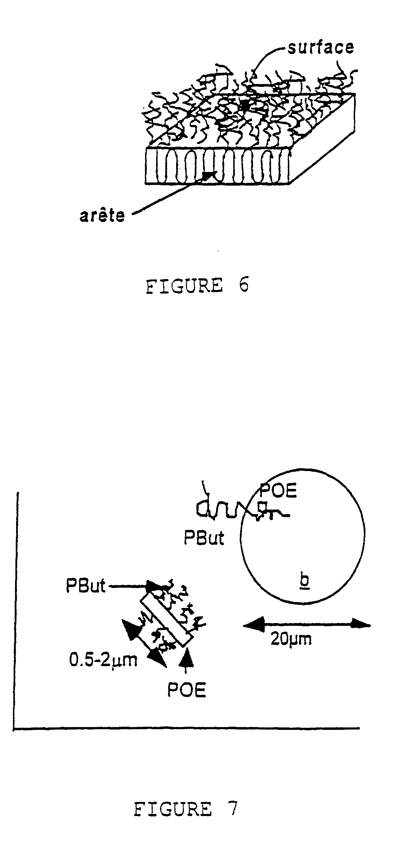

- **-** des micelles, formées d'un "coeur" de POE et d'une "couronne" de PBut. Leur présence a été mise en évidence par spectroscopie à corrélation de photons. Ces structures ont une activité tensioactive et permettent de favoriser ultérieurement la dispersion de la phase B dans A.
- **-** des agrégats rigides de forme parallélépipédique et de dimensions comprises entre 0.5 et 2 μm, formés d'une partie centrale de POE et de "cheveux" de PBut ont été mis en évidence par microscopie optique et par microscopie à force atomique. Ils peuvent être représentés à la figure 6.

La formation de telles structures est expliquée essentiellement par le caractère cristallin de la séquence POE.

Les agrégats représentent 60 % en masse du copolymère initial, et peuvent être au besoin séparés des micelles par centrifugation, puis repris dans du méthylcyclohexane de telle sorte à y former une solution de concentration donnée.

- Formation de l'émulsion de B dans A :

A 100 grammes de la solution de SEBS est rajouté 6 grammes de la solution de copolymère contenant les micelles (*copolymère dispersant*) et les agrégats (*stabilisant particulaire*) dans le méthylcyclohexane (solution à 10 % en masse). Cette solution contient 0.24 gramme de copolymère sous la forme de *copolymère dispersant* (micelles) et 0.36 gramme sous la forme de *stabilisant particulaire* (agrégats). En variante, il est possible d'ajouter séparément les micelles puis le *stabilisant particulaire*, ce dernier ayant été séparé des micelles puis repris dans le méthylcyclohexane.

L'ensemble est homogénéisé avant ajout, sous agitation, de 12 grammes de phase B.

Les fractions massiques de la phase B et de chacun des constituants sont résumées ci-dessous :

$$\phi_B = 0.102$$

$$\phi_{CD} = 0.020$$

$$\phi_{SP} = 0.029$$

$$\phi_{ATS} = 0$$

Après 15 minutes d'agitation sous conditions peu cisaillantes et à 25°C, on obtient une émulsion de diamètre modal de 20 μm.

La structure schématique de cette émulsion est représentée à la figure 7.

La stabilité de cette émulsion, c'est à dire l'absence de phénomènes de coalescence et de floculation, a été démontrée à l'aide du TURBISCAN MA 1000.

En variante, le *stabilisant particulaire* peut être ajouté, sous faible agitation, après émulsification de la phase B dans la phase A, le copolymère sous forme de micelle servant de dispersant.

Les caractéristiques de l'émulsion sont inchangées.

En remplaçant le *stabilisant particulaire* par une quantité équivalente de copolymère sous forme de micelles, l'émulsion formée a un diamètre modal de 7 μm et n'est pas stable, comme indiqué par l'étude au TURBISCAN MA1000 (diminution du signal de rétrodiffusion au cours du temps).

**Exemple 3 : Émulsion préparée en présence d'un copolymère dibloc poly (4-tertiobutyl styrène)-polyoxyéthy-lène, noté P<sup>t</sup>BuSt-POE (*copolymère dispersant*) et d'un *stabilisant particulaire* organique formé in situ à partir de ce copolymère dibloc**

Dans cet exemple, le copolymère PBut-POE est remplacé par un copolymère dibloc poly(4-tertiobutylstyrène)-polyoxyéthylène noté P$^t$BuSt-POE, contenant 45 % en masse de POE (détermination par RMN $^1$H), et de masse molaire totale de 7700 Daltons.

Ce copolymère est mis en solution dans le méthylcyclohexane (solution à 10 % en masse, formée à partir de 10 grammes de copolymère dans 90 grammes de solvant a), et y forme un mélange de micelles et d'agrégats, ces derniers étant de forme parallélépipédique et composés d'une structure centrale de POE cristallisé et de "cheveux" de P$^t$BuSt (voir figure 6). Leur dimension, évaluée par microscopie optique, est comprise entre 1 et 3 μm. Ces agrégats représentent 50% en masse du copolymère initial, et jouent dans ce cas le rôle de *stabilisant particulaire*. Les "cheveux" de P$^t$BuSt permettent la dispersion du *stabilisant particulaire* dans le solvant a, de telle sorte qu'il ne soit pas nécessaire d'utiliser d'agent de traitement de surface.

Le mode de préparation est similaire à celui décrit dans l'exemple 2, à savoir :

- préparation d'une dissolution de SEBS contenant 15 % en masse de SEBS dans du méthylcylohexane
- préparation de la phase B par mélange de Bardac et de PEG400 en proportion massique 3:7

- Formation de l'émulsion de B dans A :

A 100 grammes de solution de SEBS, est rajouté 6 grammes de la solution contenant les micelles et les agrégats (*stabilisant particulaire*) dans le méthylcyclohexane (solution à 10 %).

L'ensemble est homogénéisé avant ajout, sous agitation, de 12 grammes de phase B.

Les fractions massiques de la phase B et de chacun des constituants sont résumées ci-dessous :

$$\phi_B = 0.102$$

$$\phi_{CD} = 0.024$$

$$\phi_{SP} = 0.024$$

$$\phi_{ATS} = 0$$

Après 15 minutes d'agitation sous conditions peu cisaillantes et à 25°C on obtient une émulsion stable de diamètre modal de 30 μm. Le suivi de la stabilité de l'émulsion au moyen du Turbiscan MA1000 met en évidence l'absence de phénomènes de floculation ou de coalescence.

**Exemple 4 : Émulsion préparée en présence d'un copolymère dibloc polybutadiène hydrogéné-polyoxyéthylène, noté HPBut-POE (*copolymère dispersant*) et d'un *stabilisant particulaire* organique formé in situ à partir de ce copolymère dibloc : Suivi du diamètre de l'émulsion en fonction du temps**

Cet exemple est similaire aux exemples 2 et 3 décrits ci-dessus. Le copolymère dispersant est un copolymère dibloc HPBut-POE, contenant 50 % en masse de POE (détermination par RMN $^1$H), et de masse molaire totale de 8300 Daltons.

Ce copolymère est mis en solution dans le méthylcyclohexane (solution à 10 % en masse, formée à partir de 10 grammes de copolymère dans 90 grammes de solvant a), et y forme un mélange de micelles et d'agrégats, ces derniers représentant environ 75 % en masse du copolymère initial, et jouent dans ce cas le rôle de *stabilisant particulaire*.

Le mode de préparation est similaire à celui décrit dans l'exemple 2.

- Formation de l'émulsion de B dans A :

A 100 grammes d'une solution de SEBS identique à celle utilisée dans l'exemple précédent, est rajouté 6 grammes de la solution contenant les micelles et les agrégats (*stabilisant particulaire*) dans le méthylcyclohexane (solution à 10 %). L'ensemble est homogénéisé avant ajout, sous agitation, de 12 grammes de phase B.

Les fractions massiques de la phase B et de chacun des constituants sont résumées ci-dessous :

$$\phi_B = 0.102$$

$$\phi_{CD} = 0.012$$

$$\phi_{SP} = 0.036$$

$$\phi_{ATS} = 0$$

Après 15 minutes d'agitation sous conditions peu cisaillantes et à 25°C on obtient une émulsion stable de diamètre modal de 22 μm. Le diamètre de l'émulsion a été mesuré au cours du temps. Pour ce faire, l'émulsion est conservée "en statique" dans un tube bouché hermétiquement, puis agitée lentement avant la mesure, de façon à homogénéiser l'émulsion dans tout le tube, et à prélever un échantillon représentatif.

Le diamètre modal de l'émulsion est représenté en fonction du temps dans le tableau ci-dessous :

| Durée (heures) | 0 | 2 | 6 | 9 | 24 |
|---|---|---|---|---|---|
| Diamètre modal (μm) | 23.5 | 22.5 | 22.7 | 23.0 | 23.9 |

Aux incertitudes liées à l'analyse, le diamètre des gouttelettes n'a pas évolué au cours du temps, ce qui démontre que l'émulsion n'a pas coalescé ni floculé.

**Exemple 5: Emulsion préparée en présence d'un copolymère greffé (*copolymère dispersant*) et d'un *stabilisant particulaire* minéral de type silice.**

Dans cet exemple, le *copolymère dispersant* est un copolymère greffé de type polyéthersiloxane commercialisé par la société GOLDSCHMIDT A.G. sous le nom TEGOPREN 5840.

Le *stabilisant particulaire* est de la silice commercialisée par la société DEGUSSA. Ce composé peut être organisé soit sous la forme de particules primaires (sphères de diamètre 12 nm, selon DEGUSSA), soit sous la forme d'associats de ces particules, de structure moins définie et de dimension plus élevée. Enfin, cette silice a subi un traitement chimique chez le fournisseur afin de la rendre dispersable en solvant aliphatique.

Par conséquent, ce produit sera utilisé tel quel, sans ajout d'autre agent de traitement de surface.

- Préparation de la phase continue (phase A) :

La phase A est constituée d'une solution de SEBS KRATON G1652 à 15% dans le méthylcyclohexane.

La phase B est identique à celle utilisée dans l'exemple 2.

- Formation de l'émulsion de B dans A :

A 100 grammes de la solution de SEBS est rajouté 2 grammes de TEGOPREN 5840 puis, sous agitation, 1 gramme de silice (*stabilisant particulaire*).

A cette solution est ensuite ajouté, sous agitation peu cisaillante, 20 grammes de phase B.

Les fractions massiques de la phase B et de chacun des constituants sont résumées ci-dessous :

$$\phi_B = 0.167$$

$$\phi_{CD} = 0.090$$

$$\phi_{SP}=0.047$$

$$\phi_{ATS}=0 \; (*)$$

(*) : l'agent de traitement de surface est apporté directement par le fournisseur.

Après 15 minutes d'agitation sous conditions peu cisaillantes, on obtient une émulsion stable de diamètre modal de 15 μm.

De façon avantageuse, la viscosité de l'émulsion à cisaillement faible est augmentée par la présence de silice organophile, ce qui réduit la vitesse de sédimentation de la phase B.

L'analyse des profils de diffusion et de rétrodiffusion de cette émulsion au moyen du Turbiscan MA1000 démontre qu'il n'y a ni coalescence ni floculation.

**Exemple 6 : Émulsion préparée en présence d'un copolymère dibloc P<sup>t</sup>BuSt-POE (*copolymère dispersant*) et d'un *stabilisant particulaire* minéral de type argile.**

Dans cet exemple, le *copolymère dispersant* est un copolymère blocs P<sup>t</sup>BuSt-POE de caractéristiques analogues à celui utilisé dans l'exemple 3.

Après mise en solution dans le méthylcyclohexane (solution à 10 % en masse), des micelles et d'autres structures associées sont formées. Ces dernières sont séparées des micelles par centrifugation et représentent 50 % en masse du copolymère. Les micelles forment une solution à 5 % en masse dans le méthylcyclohexane.

Le *stabilisant particulaire* est une argile organophile de type CLAYTONE AF commercialisée par la société LAPORTE ABSORBANTS. Sa structure est de type lamellaire, ses dimensions étant de l'ordre du micromètre.

Comme dans l'exemple précédent, ce *stabilisant particulaire* est rendu organophile par le fournisseur par traitement chimique (fixation en surface de l'argile d'un ammonium quaternaire portant des fonctions alkyles), et sera donc utilisé tel quel.

- Préparation de la phase continue (phase A) :

La phase A est constituée d'une solution de SEBS KRATON G1652 à 15% dans le méthylcyclohexane.

La phase B est identique à celle utilisée dans les exemples 2 et 3.

- Formation de l'émulsion de B dans A :

A 100 grammes de la solution de SEBS est rajouté 20 grammes de solution de copolymère sous forme de micelles dans le méthylcyclohexane (5 % en masse) puis, sous agitation, 0.5 gramme de CLAYTONE AF (*stabilisant particulaire*).

20 grammes de phase B sont ensuite introduits sous agitation peu cisaillante.

Les fractions massiques de chacune des phases et des constituants sont résumées ci-dessous :

$$\phi_{B}=0.144$$

$$\phi_{CD}=0.05$$

$$\phi_{SP}=0.024$$

$$\phi_{ATS}=0 \; (*)$$

(*) : l'agent de traitement de surface est apporté directement par le fournisseur.

Après 15 minutes d'agitation sous conditions peu cisaillantes, on obtient une émulsion stable de diamètre modal de 15 μm.

De façon avantageuse, l'addition d'argile organophile se traduit par un accroissement de la viscosité à très faible cisaillement ou au repos. Tout comme pour l'exemple 5 précédent, l'analyse des profils de diffusion et de rétrodiffusion de cette émulsion au moyen du Turbiscan MA1000 démontre qu'il n'y a ni coalescence ni floculation.

**Exemple 7: Réalisation d'une émulsion à partir d'une solution de copolymère P$^t$BuSt-POE contenant des micelles et des agrégats, et d'un second *stabilisant particulaire* de type argile.**

Cet exemple est une variante de l'exemple 6 décrit ci-dessus.

Ici, la solution brute de copolymère, c'est-à-dire la solution conte nant les micelles et les agrégats, est introduite telle quelle dans la phase A.

Aussi, deux types de *stabilisant particulaire* sont finalement présents dans la solution : l'argile organophile, et les agrégats formés par le copolymère.

L'émulsion se prépare de façon analogue à celle décrite dans l'exemple 6, c'est-à-dire :

- par ajout à la solution de SEBS de la solution du copolymère contenant les micelles et les agrégats, puis,
- par ajout, sous agitation, de l'argile organophile,
- et enfin par ajout, sous faible cisaillement, de la phase B

Si la quantité de copolymère dispersant (soit $\phi_{CD}$) est identique à celle utilisée dans l'exemple précédent, l'émulsion a des caractéristiques (diamètre, stabilité) similaires.

Si la quantité totale de copolymère introduit est identique à celle de l'exemple 6 (dans ce cas une fraction du copolymère sous forme de micelles a été remplacée par des agrégats), l'émulsion qui est formée a un diamètre plus élevé (20-25 µm) tout en étant stable.

**Exemple 8 : Emulsion préparée en présence d'une substance chimique active x servant d'agent dispersant et d'un stabilisant particulaire organique constitué par des structures figées d'un copolymère dibloc polybutadiène hydrogéné - poly(oxyéthylène)**

Dans cet exemple, le copolymère dispersant, dont le rôle est d'abaisser la tension de surface entre la phase A et la phase B, est remplacé par une substance chimique x possédant à la fois une activité virucide et des caractéristiques tensioactives similaires à celles du copolymère. Cette substance chimique, introduite directement dans la phase B, est du nonyl phénol éthoxylé, commercialisé par la société Union Carbide sous le nom Triton X100.

Le *stabilisant particulaire* est un copolymère bloc HPBut-POE, contenant 50 % en masse de POE, de caractéristiques analogues à celui utilisé dans l'exemple 4. Ce copolymère est mis en solution dans le méthylcyclohexane (solution à 10 % en masse, formée à partir de 10 grammes de copolymère dans 90 grammes de solvant a), et y forme un mélange de micelles et d'agrégats, ces derniers représentant environ 75 % en masse du copolymère initial. Les agrégats sont séparés des micelles par centrifugation et repris dans du méthylcyclohexane de façon à y former une solution à 5 %

La préparation d'une émulsion stable et de granulométrie élevée conformément à l'invention s'effectue comme suit :

- Préparation de la phase continue (phase A) :

La phase A est constituée d'une solution de SEBS KRATON G1652 à 15 % dans le méthylcyclohexane.

- Préparation de la phase dispersée (phase B) :

On dissout, sous agitation, du Triton X100 dans du polyethylène glycol de masse molaire 400 Daltons (PEG 400) de manière à obtenir une solution à 30 % en masse en Triton X100.

- Formation de l'émulsion de B dans A :

A 100 grammes de la solution de SEBS sont ajoutés 20 grammes de solution de copolymère sous forme d'agrégats dans le méthylcyclohexane (5 % en masse), puis 20 grammes de phase B.

Les fractions massiques de chacune des phases et des constituants sont résumées ci-dessous :

$$\phi_B=0.167$$

$$\phi_x = 0.30$$

$$\phi_{SP} = 0.047$$

$$\phi_{ATS} = 0 \text{ (absence d'agent de traitement de surface).}$$

Après 15 minutes d'agitation sous conditions peu cisaillantes, on obtient une émulsion stable de diamètre modal de 25 μm.

Cet exemple confirme que, lorsque la substance chimique active x a des propriétés tensioactives similaires à celles du copolymère dispersant, cette dernière permet à elle seule, en combinaison avec le stabilisant particulaire, de favoriser la dispersion de la phase B dans la phase A.

## Exemple 9 : Préparation d'un film d'élastomère.

On évapore sous pression atmosphérique à température ambiante le solvant a à partir d'une émulsion telle définie aux exemples 2 à 8 ; on obtient ainsi un film d'élastomère renfermant sous forme de dispersion stable, des gouttelettes de solvant b, chargé en substance chimique active x, d'un diamètre ≥ 10 μm.

## Revendications

**1.** Émulsion stable d'au moins une substance chimique x dans une solution d'élastomère, apte à être utilisée pour la préparation d'un film d'élastomère, comprenant (1) une phase A comprenant un élastomère dissous dans un solvant organique a apolaire ou peu polaire, dans laquelle est dispersée (2) une phase B comprenant au moins ladite substance chimique x, en solution ou dispersée dans un solvant b polaire, non miscible avec la phase A et (3) au moins un agent dispersant sélectionné dans le groupe constitué par les copolymères à blocs ou greffés, laquelle émulsion est **caractérisée :**

- **en ce que** les gouttelettes de phase dispersée B ont un diamètre ≥ 10 μm,
- **en ce que** ladite émulsion comprend, pour la stabilisation de ladite phase dispersée B, outre au moins un *copolymère dispersant* comportant des séquences poly A, compatibles avec la phase A et des séquences poly B compatibles avec la phase B, au moins un *stabilisant particulaire* sélectionné dans le groupe constitué par des composés solides organiques de dimension comprise entre 30 nm et 10 μm ou des composés solides minéraux de dimension comprise entre 5 nm et 10 μm, dont l'état de surface est organophile,
- **en ce que** la fraction massique $\phi_B$ de phase dispersée (phase B) dans l'émulsion, exprimée par :

$$\phi_B = \frac{m_B}{m_B + m_A + m_{élastomère}}$$

est comprise entre 0,01 et 0,2,

avec $m_B$=masse de phase B (solvant b + substance chimique active x)

$m_A$= masse de solvant a

$m_{élastomère}$= masse de l'élastomère dissous dans a ;

- **en ce que** la fraction massique du copolymère à blocs ou greffé, $\phi_{CD}$, exprimée par rapport à la phase dispersée B :

$$\phi_{CD} = \frac{m_{CD}}{m_{CD} + m_B}$$

**16**

est comprise entre 0,001 et 0,3, de préférence entre 0,01 et 0,2,

avec $m_B$=masse de phase B
$m_{CD}$=masse de copolymère dispersant ; et

- **en ce que** la fraction massique de *stabilisant particulaire* (SP), $\phi_{SP}$, formé *in situ* ou ajouté en tant qu'adjuvant, exprimée par rapport à la phase dispersée B :

$$\phi_{SP} = \frac{m_{SP}}{m_{SP} + m_B}$$

est comprise entre 0,001 et 0,5,

avec $m_{SP}$= masse de *stabilisant particulaire*

$m_B$= masse de phase B.

2. Emulsion selon la revendication 1, **caractérisée en ce que** ladite fraction massique du copolymère à blocs ou greffé, $\phi_{CD,}$ est comprise entre 0,001 et 0,2, de préférence entre 0,01 et 0,1.

3. Émulsion selon la revendication 1 ou la revendication 2, **caractérisée en ce que** lorsque ledit *stabilisant particulaire* est de nature organique, il est sélectionné dans le groupe constitué par des copolymères à blocs ou greffé organophiles, identiques ou différents de ceux utilisés comme *copolymères dispersants,* aptes à former des structures d'un diamètre supérieur à 30 nm et des dérivés de la cellulose comme la microcellulose, l'amidon, ou certains polymères finement divisés, comportant à leur surface, un agent de traitement de surface apte à rendre ledit *stabilisant particulaire* organophile, la fraction massique de l'agent de traitement de surface, utilisé pour rendre la surface des particules organophile, soit $\phi_{ATS,}$ exprimée par rapport au *stabilisant particulaire* :

$$\phi_{ATS} = \frac{m_{ATS}}{m_{SP} + m_{ATS}}$$

étant comprise entre 0,001 et 0,5,

avec $m_{ATS}$=masse de l'agent de traitement de surface
$m_{SP}$= masse de *stabilisant particulaire*.

4. Emulsion selon la revendication 3, **caractérisée en ce que** ladite fraction massique de l'agent de traitement de surface, $\phi_{ATS,}$ est comprise entre 0,001 et 0,1.

5. Émulsion selon la revendication 3 ou la revendication 4, **caractérisée en ce que** lesdits copolymères à blocs ou greffé organophiles sont sélectionnés dans le groupe constitué par les copolymères contenant une séquence polyB cristallisable, comme par exemple polyoxyéthylène, poly(éthylène), polyamide ou polyester tel que poly (caprolactone) et des séquences poly A solubles dans la phase A de l'émulsion.

6. Émulsion selon la revendication 1 ou la revendication 2, **caractérisée en ce que** lorsque le *stabilisant particulaire* est de type minéral, ce dernier est choisi dans le groupe qui comprend les argiles, les silices, le talc, le kaolin ou des dérivés de ces produits et comportant à leur surface, un agent de traitement de surface apte à rendre ledit *stabilisant particulaire* organophile, la fraction massique de l'agent de traitement de surface, utilisé pour rendre la surface des particules organophile, soit $\phi_{ATS,}$ exprimée par rapport au *stabilisant particulaire* :

$$\phi_{ATS} = \frac{m_{ATS}}{m_{SP} + m_{ATS}}$$

est comprise entre 0,001 et 0,5,

avec m$_{ATS}$=masse de l'agent de traitement de surface

m$_{SP}$= masse de *stabilisant particulaire*.

**7.** Emulsion selon la revendication 6, **caractérisée en ce que** ladite fraction massique de l'agent de traitement de surface, φ$_{ATS,}$ est comprise entre 0,001 et 0,1.

**8.** Émulsion selon l'une quelconque des revendications 3 à 7, **caractérisée en ce que** l'agent de traitement de surface est sélectionné dans le groupe constitué par des copolymères à blocs ou greffé contenant au moins une séquence polyA', soluble dans le solvant a, et une séquence polyB' capable de s'adsorber ou de s'ancrer à la surface du *stabilisant particulaire*, lesdites séquences poly A' et poly B' étant identiques ou différentes des séquences poly A et poly B et des oligomères fonctionnalisés de type polyA'-F, dans lesquels F est une fonction chimique capable de s'adsorber sur le *stabilisant particulaire*.

**9.** Émulsion selon la revendication 8, **caractérisée en ce que** les séquences polyA' sont choisies parmi les polymères solubles dans le solvant apolaire ou peu polaire a, comme par exemple dans le groupe qui comprend les polydiènes, les polyoléfines, les polyéthers ou les silicones, tels le polyisoprène, le polybutadiène, le polyisobutène, le polybutadiène hydrogéné ou le polyisoprène hydrogéné, le poly(4-tertiobutylstyrène), le polyoxypropylène, le polyoxybutylène, le polydiméthylsiloxane, le poly(méthacrylate de 2-ethyle hexyl), le poly(méthacrylate de lauryle) miscibles avec une solution d'élastomère dans un solvant a, et les séquences poly B' sont choisies parmi les polymères capables de s'adsorber à la surface du *stabilisant particulaire*, et est choisi dans le groupe qui comprend le polyoxyéthylène, la polyvinylpyrrolidone, les polyacides acryliques, le poly(alcool vinylique) et le poly(vinylpyridine) quaternisé ou non.

**10.** Émulsion selon la revendication 9, **caractérisée en ce que** les proportions de séquence polyA', exprimées en masse par rapport à la somme des séquences polyA'+polyB', sont comprises entre 10 et 90 %, de préférence entre 20 et 80 % et les proportions de séquences polyB' sont comprises entre 90 et 10 %.

**11.** Émulsion selon la revendication 9 ou la revendication 10, **caractérisée en ce que** les masses molaires des séquences polyA' et polyB' sont comprises entre 150 et 200 000 Daltons.

**12.** Émulsion selon la revendication 8, **caractérisée en ce que** F est sélectionné dans le groupe constitué par les fonctions acides, amines ou alcool et les groupements apte à réagir chimiquement avec la surface du *stabilisant particulaire*, comme par exemple par des groupements époxy, isocyanate ou aziridine.

**13.** Émulsion selon l'une quelconque des revendications 1 à 12, **caractérisée en ce que** le copolymère à bloc dispersant est sélectionné parmi les copolymères di-bloc, de type polyA-bloc-polyB, les copolymères tribloc de type polyB-bloc-polyA-bloc-polyB (BAB), de type polyA-bloc-polyB-bloc-polyA (ABA), de type polyA-bloc-polyB-bloc-polyC (ABC) ou polyA-bloc-polyC-bloc-polyB (ACB), ou plus généralement parmi les composés multiblocs contenant des séquences polyA, polyB et polyC et le copolymère du type greffé dispersant est sélectionné parmi les composés de type polyA-greffé-polyB, polyB-greffé-polyA, de type polyA-greffé-polyB et polyC ou de type polyC-greffé-polyA et polyB.

**14.** Émulsion selon la revendication 13, **caractérisée en ce que** les proportions de séquence polyA, exprimées en masse par rapport à la somme des séquences polyA+polyB, sont comprises entre 10 et 90 %, et les proportions de séquences polyB sont comprises entre 90 et 10 %, et les proportions massiques de séquences polyC sont comprises entre 0 et 50 % par rapport à l'ensemble des séquences.

**15.** Émulsion selon la revendication 13 ou la revendication 14, **caractérisée en ce que** les masses molaires des séquences poly A, poly B et poly C sont comprises entre 1 000 et 500 000 Daltons.

**16.** Émulsion selon l'une quelconque des revendications 13 à 15, **caractérisée en ce que** les séquences poly A, compatibles avec le solvant a, sont sélectionnées dans le groupe constitué par les polydiènes, les polyoléfines, les polyéthers ou les silicones, tels le polyisoprène, le polybutadiène, le polyisobutène, le polybutadiène hydrogéné ou le polyisoprène hydrogéné, le poly(4-tertiobutylstyrène), le polyoxypropylène, le polyoxybutylène, le polydiméthylsiloxane, le poly(méthacrylate de 2-ethyle hexyl), le poly(méthacrylate de lauryle) miscibles avec une solution d'élastomère dans un solvant a, les séquences polyB, compatibles avec le solvant b, sont sélectionnées dans le groupe constitué par le polyoxyéthylène, la polyvinylpyrrolidone, les polyacides acryliques, le poly(alcool vinylique) et le poly(vinylpyridine) quaternisé et les séquences polyC, compatibles soit avec le solvant $\underline{a}$, soit avec le solvant $\underline{b}$, soit non compatible avec les solvants $\underline{a}$ et $\underline{b}$, sont sélectionnées dans le groupe constitué par les polymères acryliques ou vinyliques tels le poly(méthacrylate de méthyle) ou le polystyrène.

**17.** Émulsion selon l'une quelconque des revendications 1 à 16, **caractérisée en ce que** l'élastomère est sélectionné dans le groupe constitué par le polybutadiène, le polyisoprène, le polychloroprène, les copolymères SBR (*Styrene Butadiene Rubber*), NBR (*Nitrile Butadiene Rubber*), SBS (*Styrene Butadiene Styrene*), SIS (*Styrene Isoprene Styrene*), SEBS (*Styrene Ethylene-co-Butylene Styrene*) seul ou en mélange avec un ou plusieurs plastifiants ou flexibilisants.

**18.** Émulsion selon l'une quelconque des revendications 1 à 16, **caractérisée en ce que** le solvant $\underline{a}$ est choisi notamment parmi les hydrocarbures aliphatiques, aromatiques et alicycliques, par exemple le méthylcyclohexane, le toluène, l'heptane, ou un mélange de ceux-ci.

**19.** Émulsion selon l'une quelconque des revendications 1 à 16, **caractérisée en ce que** la substance chimique active $\underline{x}$ est sélectionnée parmi les composés capables de provoquer une dénaturation quasi-instantanée des protéines par simple contact, soit par réaction chimique, soit par effet physico-chimique tel qu'une modification de la tension de surface

**20.** Émulsion selon la revendication 19, **caractérisée en ce que** ladite substance chimique active $\underline{x}$ est choisie parmi les biocides.

**21.** Émulsion selon la revendication 19 ou la revendication 20, **caractérisée en ce que** ledit biocide est sélectionné parmi les ammonium quaternaires, de préférence du chlorure de diméthyldidécylammonium, des biguanides, le phtalaldéhyde, des dérivés phénoliques ou benzyliques, le formol, des tensioactifs non-ioniques comportant au moins une séquence polyoxyéthylene, l'hexamidine, des composés iodés de polyvinylpyrrolidone, des tensioactifs non-ioniques à activité virucide, les bichromates et hypochlorites de sodium et de potassium, utilisés seuls ou en mélange.

**22.** Émulsion selon l'une quelconque des revendications 1 à 21, **caractérisée en ce que** la fraction massique de substance chimique active $\underline{x}$, soit $\phi_x$, est exprimée par

$$\phi_x = \frac{m_x}{m_x + m_b} = \frac{m_x}{m_B}$$

et est comprise entre 0,01 et 0,7, de préférence entre 0,1 et 0,4,

avec $m_x$ = masse de substance chimique active $\underline{x}$

$m_b$ = masse de solvant $\underline{b}$

$m_B$ = masse de phase dispersée B.

**23.** Émulsion selon l'une quelconque des revendications 1 à 22, **caractérisée en ce que** le solvant $\underline{b}$ est non miscible avec le solvant $\underline{a}$ et est sélectionné parmi les polyols, et de préférence la glycérine, l'éthylène glycol et les polyéthylènes glycols liquides à température ambiante et de masse molaire comprise entre 62 (éthylène glycol) et 750 Daltons (PEG 750) et tout autre composé non miscible avec le solvant $\underline{a}$ comme par exemple l'eau, la diméthylsulfoxyde, la formamide ou l'éthanolamine ou les mélanges de tels solvants.

**24.** Émulsion selon l'une quelconque des revendications 1 à 23, **caractérisée en ce qu'**elle comprend plusieurs *stabilisants particulaires*.

**25.** Émulsion stable d'au moins une substance chimique $\underline{x}$ dans une solution d'élastomère, apte à être utilisée pour la préparation d'un film d'élastomère, comprenant (1) une phase A comprenant un élastomère dissous dans un solvant organique $\underline{a}$ apolaire ou peu polaire, dans laquelle est dispersée (2) une phase B comprenant au moins ladite substance chimique $\underline{x}$, en solution ou dispersée dans un solvant $\underline{b}$ polaire, non miscible avec la phase A , laquelle émulsion est **caractérisée :**

- **en ce que** les gouttelettes de phase dispersée B ont un diamètre $\geq 10\ \mu m$,
- **en ce que** ladite émulsion comprend, pour la stabilisation de ladite phase dispersée B, **(1)** au moins une substance chimique $\underline{x}$, cette dernière ayant des propriétés tensioactives et jouant le rôle d'agent dispersant, ladite substance chimique $\underline{x}$ étant sélectionnée dans le groupe constitué par les ammoniums quaternaires, les tensioactifs non-ioniques comportant au moins une séquence polyoxyéthylène et les tensioactifs non-ioniques à activité virucide, et **(2)** au moins un *stabilisant particulaire* tel que défini dans la revendication 1, de préférence formé à partir d'un copolymère à blocs ou greffé,
- **en ce que** la fraction massique $\phi_B$ de phase dispersée (phase B) dans l'émulsion, exprimée par :

$$\phi_B = \frac{m_B}{m_B + m_A + m_{élastomère}}$$

est comprise entre 0,01 et 0,2,

avec $m_B$=masse de phase B (solvant $\underline{b}$ + substance chimique active $\underline{x}$)

$m_A$= masse de solvant $\underline{a}$

$m_{élastomère}$ = masse de l'élastomère dissous dans $\underline{a}$ ;

- **en ce que** la fraction massique de la substance chimique active $\underline{x}$, soit $\phi_x$ , exprimée par

$$\phi_x = \frac{m_x}{m_x + m_b} = \frac{m_x}{m_B}$$

est comprise entre 0,01 et 0,7, de préférence entre 0,1 et 0,4,

avec $m_x$= masse de substance chimique active $\underline{x}$

$m_b$= masse de solvant $\underline{b}$

$m_B$= masse de phase dispersée B,

- **en ce que** la fraction massique de *stabilisant particulaire* (SP), $\phi_{SP}$, formé *in situ* ou ajouté en tant qu'adjuvant, exprimée par rapport à la phase dispersée B :

$$\phi_{SP} = \frac{m_{SP}}{m_{SP} + m_B}$$

est comprise entre 0,001 et 0,5,

avec $m_{SP}$= masse de *stabilisant particulaire*

$m_B$= masse de phase B.

**26.** Emulsion selon la revendication 25, **caractérisée en ce que** ledit élastomère, ledit solvant $\underline{a}$, ledit solvant $\underline{b}$ et ledit stabilisant particulaire sont tels que définis dans les revendications 3 à 12, 17, 18, 23 et 24.

**27.** Procédé de préparation d'une émulsion stable selon l'une quelconque des revendications 1 à 24, **caractérisé en ce qu'**il comprend dans le cas où le *stabilisant particulaire* est ajouté <u>avant</u> la formation de l'émulsion :

- la préparation d'une phase A par dissolution de l'élastomère dans un solvant organique $\underline{a}$ (phase A : solution d'élastomère dans le solvant organique $\underline{a}$),
- la préparation d'une phase B, par mélange d'une substance chimique active $\underline{x}$ dans un solvant organique $\underline{b}$ non miscible avec la phase A (phase B : solution ou dispersion de substance active dans un solvant organique b non miscible avec la phase A),

- l'addition à la phase A ou à la phase B, dans les proportions telles que définies ci-dessus, d'un copolymère à blocs ou greffé servant essentiellement de *copolymère dispersant* et d'un *stabilisant particulaire* organophile ou rendu organophile,
- la dispersion de la phase B dans la phase A pour l'obtention d'une émulsion dans laquelle la phase A constitue la phase continue et la phase B, la phase dispersée.

28. Procédé de préparation d'une émulsion stable selon la revendication 25 ou la revendication 26, **caractérisé en ce qu'**il comprend dans le cas où le *stabilisant particulaire* est ajouté <u>avant</u> la formation de l'émulsion :

- la préparation d'une phase A par dissolution de l'élastomère dans un solvant organique <u>a</u> (phase A : solution d'élastomère dans le solvant organique <u>a</u>),
- la préparation d'une phase B, par mélange d'une substance chimique active <u>x</u>, telle que définie dans la revendication 25, dans un solvant organique <u>b</u> non miscible avec la phase A (phase B : solution ou dispersion de substance active dans un solvant organique <u>b</u> non miscible avec la phase A),
- l'addition à la phase A ou à la phase B, dans les proportions telles que définies ci-dessus, d'un *stabilisant particulaire* organophile ou rendu organophile, de préférence formé à partir d'un copolymère à blocs ou greffé,
- la dispersion de la phase B dans la phase A pour l'obtention d'une émulsion dans laquelle la phase A constitue la phase continue et la phase B, la phase dispersée.

29. Procédé de préparation d'une émulsion stable selon l'une quelconque des revendications 1 à 24, **caractérisé en ce qu'**il comprend dans le cas où le *stabilisant particulaire* est ajouté <u>pendant</u> ou <u>après</u> la formation de l'émulsion :

- la préparation d'une phase A par dissolution de l'élastomère dans un solvant organique <u>a</u> (phase A : solution d'élastomère dans le solvant organique <u>a</u>).
- la préparation d'une phase B, par mélange d'une substance chimique active <u>x</u> dans un solvant organique <u>b</u> non miscible avec la phase A (phase B : solution ou dispersion de substance active dans un solvant organique <u>b</u> non miscible avec la phase A),
- l'addition à la phase A ou à la phase B, dans des proportions telles que définies ci-dessus d'un copolymère à blocs ou greffé servant essentiellement de *copolymère dispersant*,
- la dispersion de la phase B dans la phase A pour l'obtention d'une émulsion dans laquelle la phase A constitue la phase continue et la phase B, la phase dispersée, et l'ajout simultané ou non, sous agitation, du *stabilisant particulaire* organophile ou rendu organophile.

30. Procédé de préparation d'une émulsion stable selon la revendication 25 ou la revendication 26, **caractérisé en ce qu'**il comprend dans le cas où le *stabilisant particulaire* est ajouté <u>pendant</u> ou <u>après</u> la formation de l'émulsion :

- la préparation d'une phase A par dissolution de l'élastomère dans un solvant organique a (phase A : solution d'élastomère dans le solvant organique <u>a</u>),
- la préparation d'une phase B, par mélange d'une substance chimique active <u>x</u>, telle que définie dans la revendication 25, dans un solvant organique <u>b</u> non miscible avec la phase A (phase B : solution ou dispersion de substance active dans un solvant organique <u>b</u> non miscible avec la phase A),
- la dispersion de la phase B dans la phase A pour l'obtention d'une émulsion dans laquelle la phase A constitue la phase continue et la phase B, la phase dispersée, et l'ajout simultané ou non, sous agitation, du *stabilisant particulaire* organophile ou rendu organophile, de préférence formé à partir d'un copolymère à blocs ou greffé.

31. Film d'élastomère, **caractérisé en ce qu'**il est obtenu par évaporation du solvant <u>a</u> d'une émulsion selon l'une quelconque des revendications 1 à 26.

32. Procédé de préparation du film d'élastomère selon la revendication 31, **caractérisé en ce qu'**il comprend :

(a) la préparation d'une émulsion selon l'une quelconque des revendications 1 à 26, et
(b) l'évaporation du solvant organique <u>a</u>, pour l'obtention d'un film d'élastomère renfermant sous forme de dispersion stable, des gouttelettes de solvant <u>b</u>, chargé en substance chimique active.

33. Application du film d'élastomère selon la revendication 31 en tant que revêtement de supports.

34. Gant, **caractérisé en ce qu'**il est comporte un film d'élastomère selon la revendication 31.

**35.** Doigtier, **caractérisé en ce qu'**il comporte un film d'élastomère selon la revendication 31.

**36.** Préservatif, **caractérisé en ce qu'**il comporte un film d'élastomère selon la revendication 31.

**37.** Pansement, **caractérisé en ce qu'**il comporte un film d'élastomère selon la revendication 31.

**Claims**

**1.** Stable emulsion of at least one chemical substance x in an elastomer solution, able to be used for preparing an elastomer film, comprising (1) a phase A comprising an elastomer dissolved in a non-polar or only slightly polar organic solvent $\underline{a}$, in which there is dispersed (2) a phase B comprising at least the said chemical structure $\underline{x}$, in solution or dispersed in a polar solvent $\underline{b}$, not miscible with the phase A, and (3) at least one dispersant selected from the group consisting of block or grafted copolymers, which emulsion is **characterised:**

- **in that** the droplets of the dispersed phase B have a diameter $\geq 10$ µm,

- **in that** the said emulsion comprises, for stabilising the said dispersed phase B, in addition to at least one *dispersing copolymer* comprising polyA sequences compatible with phase A and polyB sequences compatible with phase B, at least one *particulate stabiliser* selected from the group consisting of organic solid compounds with a dimension of between 30 nm and 10 µm or mineral solid compounds with a dimension of between 5 nm and 10 µm, whose surface state is organophilic,

- **in that** the fraction by weight $\phi_B$ of the dispersed phase (phase B) in the emulsion, expressed by:

$$\phi_B = \frac{m_B}{m_B + m_A + m_{elastomer}}$$

is between 0.01 and 0.2,

with $m_B$ = mass of phase B (solvent $\underline{b}$ + active chemical substance $\underline{x}$)
$m_A$ = mass of solvent $\underline{a}$
$m_{elastomer}$ = mass of elastomer dissolved in $\underline{a}$;

- **in that** the fraction by weight of the block or grafted polymer, $\phi_{CD}$, expressed with respect to the dispersed phase B:

$$\phi_{CD} = \frac{m_{CD}}{m_{CD} + m_B}$$

is between 0.001 and 0.3, preferably between 0.01 and 0.2,

with $m_B$ = mass of phase B
$m_{CD}$ = mass of dispersing copolymer; and

- **in that** the fraction by weight of *particulate stabiliser* (SP), $\phi_{SP}$, formed *in situ* or added as adjuvant, expressed with respect to the dispersed phase $\underline{B}$:

$$\phi_{SP} = \frac{m_{SP}}{m_{SP} + m_B}$$

is between 0.001 and 0.5,

with $m_{SP}$ = mass of *particulate stabiliser*
$m_B$ = mass of phase B.

2. Emulsion according to Claim 1, **characterised in that** the said fraction by weight of the block or grafted copolymer, $\phi_{CD}$ is between 0.001 and 0.2, preferably between 0.01 and 0.1.

3. Emulsion according to Claim 1 or Claim 2, **characterised in that**, when the said *particulate stabiliser* is of an organic nature, it is selected from the group consisting of organophilic block or grafted copolymers, identical to or different from those used as *dispersing copolymers,* able to form structures with a diameter greater than 30 nm and derivatives of cellulose such as microcellulose, starch or certain finely divided polymers, having at their surface a surface treatment agent able to make the said *particulate stabiliser* organophilic, the fraction by weight of the surface treatment agent used for making the surface of the particles organophilic, that is to say $\phi_{ATS}$, expressed with respect to the *particulate stabiliser*:

$$\phi_{ATS} = \frac{m_{ATS}}{m_{SP} + m_{ATS}}$$

being between 0.001 and 0.5,

with $m_{ATS}$ = mass of the surface treatment agent
$m_{SP}$ = mass of *particulate stabiliser.*

4. Emulsion according to Claim 3, **characterised in that** the said fraction by weight of the surface treatment agent, $\phi_{ATS}$, is between 0.001 and 0.1.

5. Emulsion according to Claim 3 or Claim 4, **characterised in that** the said organophilic block or grafted copolymers are selected from the group consisting of polymers containing a crystallisable polyB sequence, such as for example polyoxyethylene, polyethylene, polyamide or polyester such as polycaprolactone, and polyA sequences soluble in phase A of the emulsion.

6. Emulsion according to Claim 1 or Claim 2, **characterised in that**, when the *particulate stabiliser* is of the mineral type, the latter is chosen from the group which comprises clays, silica, talc, kaolin or derivatives of these products and comprising at their surface a surface treatment agent able to make the said *particulate stabiliser* organophilic, the fraction by weight of the surface treatment agent used for making the surface of the particles organophilic, that is to say $\phi_{ATS}$, expressed with respect to the *particulate stabiliser:*

$$\phi_{ATS} = \frac{m_{ATS}}{m_{SP} + m_{ATS}}$$

is between 0.001 and 0.5,

with $m_{ATS}$ = mass of the surface treatment agent
$m_{SP}$ = mass of *particulate stabiliser*.

7. Emulsion according to Claim 6, **characterised in that** the said fraction by weight of the surface treatment agent, $\phi_{ATS}$, is between 0.001 and 0.1.

8. Emulsion according to any one of Claims 3 to 7, **characterised in that** the surface treatment agent is selected from the group consisting of block or grafted copolymers containing at least one polyA' sequence, soluble in the solvent a, and a polyB' sequence capable of being absorbed or anchoring on the surface of the *particulate stabiliser,* the said polyA' and polyB' sequences being identical or different from the polyA and polyB sequences and the functionalised oligomers of type polyA'-F, in which F is a chemical function capable of being absorbed on the particulate stabiliser.

9. Emulsion according to Claim 8, **characterised in that** the polyA' sequences are chosen from amongst polymers soluble in the non-polar or only slightly polar solvent a, such as for example from the group which comprises polydienes, polyolefins, polyethers or silicones, such as polyisoprene, polybutadiene, polyisobutene, hydrogenated polybutadiene or hydrogenated polyisoprene, poly(4-tert-butylstyrene), polyoxypropylene, polyoxybutylene, polydimethylsiloxane, 2-ethylhexyl polymethacrylate or lauryl polymethacrylate all miscible with a solution of elastomer

EP 0 981 573 B1

in a solvent $\underline{a}$, and the polyB' sequences chosen from amongst the polymers capable of being absorbed on the surface of the *particulate stabiliser,* and is chosen from the group which comprises polyoxyethylene, polyvinylpyrrolidone, the acrylic polyacids, polyvinyl alcohol and polyvinylpyridine, quaternised or not.

10. Emulsion according to Claim 9, **characterised in that** the proportions of polyA' sequence, expressed by weight with respect to the sum of the polyA'+polyB' sequences, are between 10 and 90%, preferably between 20 and 80%, and the proportions of polyB' sequences are between 90 and 10%.

11. Emulsion according to Claim 9 or Claim 10, **characterised in that** the molar masses of the polyA' and polyB' sequences are between 150 and 200,000 daltons.

12. Emulsion according to Claim 8, **characterised in that** F is selected from the group consisting of acid, amine or alcohol functions and the groups able to react chemically with the surface of the *particulate stabiliser,* such as for example epoxy, isocyanate or aziridine groups.

13. Emulsion according to any one of Claims 1 to 12, **characterised in that** the dispersing block copolymer is selected from amongst the di-block copolymers, of the polyA-block-polyB type, the triblock copolymers of the polyB-block-polyA-block-polyB (BAB) type, of the polyA-block-polyB-block-polyA (ABA) type, of the polyA-block-polyB-block-polyC (ABC) type or polyA-block-polyC-block-polyB (ACB), or more generally from amongst the multiblock compounds containing polyA, polyB and polyC sequences, and the copolymer of the dispersing grafted type is selected from amongst the compounds of the polyA-graft-polyB, polyB-graft-polyA, of the polyA-graft-polyB and polyC type or of the polyC-graft-polyA and polyB type.

14. Emulsion according to Claim 15, **characterised in that** the proportions of polyA sequence, expressed by weight with respect to the sum of the polyA+polyB sequences, are between 10% and 90%, and the proportions of polyB sequences are between 90% and 10%, and the proportions by weight of polyC sequences are between 0 and 50% with respect to all the sequences.

15. Emulsion according to Claim 13 or Claim 14, **characterised in that** the molar masses of the polyA, polyB and polyC sequences are between 1000 and 500,000 daltons.

16. Emulsion according to any one of Claims 13 to 15, **characterised in that** the polyA sequences, compatible with solvent $\underline{a}$, are selected from the group consisting of polydienes, polyolefins, polyethers or silicones, such as polyisoprene, polybutadiene, polyisobutene, hydrogenated polybutadiene or hydrogenated polyisoprene, poly(4-tert-butylstyrene), polyoxypropylene, polyoxybutylene, polydimethylsiloxane, 2-ethylhexyl polymethacrylate or lauryl polymethacrylate all miscible with a solution of elastomer in a solvent $\underline{a}$, the polyB sequences, compatible with the solvent $\underline{b}$, are selected from the group consisting of polyoxyethylene, polyvinylpyrrolidone, the acrylic polyacids, polyvinyl alcohol and quaternised polyvinylpyridine, and the polyC sequences, compatible either with solvent $\underline{a}$ or with solvent $\underline{b}$, that is to say not compatible with solvents $\underline{a}$ and $\underline{b}$, are selected from the group consisting of the acrylic or vinyl polymers such as polymethyl methacrylate or polystyrene.

17. Emulsion according to any one of Claims 1 to 16, **characterised in that** the elastomer is selected from the group consisting of polybutadiene, polyisoprene, polychloroprene, the copolymers SBR (*Styrene Butadiene Rubber*), NBR (*Nitrile Butadiene Rubber*), SBS (*Styrene Butadiene Styrene*), SIS (*Styrene Isoprene Styrene*) or SEBS (*Styrene Ethylene-co-Butylene Styrene*) alone or in a mixture with one or more plasticisers or flexibility agents.

18. Emulsion according to any one of Claims 1 to 16, **characterised in that** the solvent $\underline{a}$ is chosen in particular from amongst aliphatic, aromatic and alicyclic hydrocarbons, for example methylcyclohexane, toluene, heptane or a mixture of these.

19. Emulsion according to any one of Claims 1 to 16, **characterised in that** the active chemical substance $\underline{x}$ is selected from amongst the compounds capable of causing an almost instantaneous denaturation of the proteins by simple contact, either by chemical reaction, or by physico-chemical effect such as a modification of the surface tension.

20. Emulsion according to Claim 19, **characterised in that** the said active chemical substance $\underline{x}$ is chosen from amongst biocides.

21. Emulsion according to Claim 19 or Claim 20, **characterised in that** the said biocide is selected from amongst

24

quaternary ammoniums, preferably dimethyldidecylammonium chloride, biguanides, phthalaldehyde, phenolic or benzyl derivatives, formalin, non-ionic surfactants comprising at least one polyoxyethylene sequence, hexamidine, iodised compounds of polyvinylpyrrolidone, non-ionic surfactants with virucidal activity, sodium and potassium bichromates and hypochlorites, used alone or in a mixture.

22. Emulsion according to any one of Claims 1 to 21, **characterised in that** the fraction by weight of active chemical substance $\underline{x}$, that is to say $\phi_x$, is expressed by

$$\phi_x = \frac{m_x}{m_x + m_b} = \frac{m_x}{m_B}$$

and is between 0.01 and 0.7, preferably between 0.1 and 0.4,

with $m_x$ = mass of active chemical substance $\underline{x}$
$m_b$ = mass of solvent $\underline{b}$
$m_B$ = mass of dispersed phase B.

23. Emulsion according to any one of Claims 1 to 22, **characterised in that** the solvent $\underline{b}$ is non-miscible with solvent $\underline{a}$ and is selected from amongst polyols, and preferably glycerine, ethylene glycol and the polyethylene glycols liquid at ambient temperature and with a molar mass of between 62 (ethylene glycol) and 750 daltons (PEG 750) and any other compound not miscible with solvent $\underline{a}$ such as for example water, dimethylsulphoxide, formamide or ethanolamine or mixtures of such solvents.

24. Emulsion according to any one of Claims 1 to 23, **characterised in that** it comprises several *particulate stabilisers*.

25. Stable emulsion of at least one chemical substance $\underline{x}$ in an elastomer solution, able to be used for preparing an elastomer film, comprising (1) a phase A comprising an elastomer dissolved in a non-polar or only slightly polar organic solvent $\underline{a}$, in which there is dispersed (2) a phase B comprising at least the said chemical substance $\underline{x}$, in solution or dispersed in a polar solvent $\underline{b}$, not miscible with phase A, which emulsion is **characterised:**

  • **in that** the droplets of the dispersed phase B have a diameter $\geq 10$ µm,

  • **in that** the said emulsion comprises, for stabilising the said dispersed phase B, (1) at least one chemical substance $\underline{x}$, the latter having surface active properties and fulfilling the role of dispersing agent, the said chemical substance $\underline{x}$ being selected from the group consisting of quaternary ammoniums, non-ionic surfactants comprising at least one polyoxyethylene sequence and the non-ionic surfactants with virucidal activity, and (2) at least one *particulate stabiliser* as defined in Claim 1, preferably formed from a block or grafted copolymer,

  • **in that** the fraction by weight $\phi_B$ of the dispersed phase (phase B) in the emulsion, expressed by:

$$\phi_B = \frac{m_B}{m_B + m_A + m_{elastomer}}$$

is between 0.01 and 0.2,
with $m_B$ = mass of phase B (solvent $\underline{b}$ + active chemical substance $\underline{x}$)

$m_A$ = mass of solvent $\underline{a}$
$m_{elastomer}$ = mass of elastomer dissolved in $\underline{a}$;

  • **in that** the fraction by weight of the active chemical substance $\underline{x}$, that is to say $\phi_x$, expressed by

$$\phi_x = \frac{m_x}{m_x + m_b} = \frac{m_x}{m_B}$$

is between 0.01 and 0.7, preferably between 0.1 and 0.4,

with $m_x$ = mass of active chemical substance $\underline{x}$
$m_b$ = mass of solvent $\underline{b}$
$m_B$ = mass of dispersed phase B,

- **in that** the fraction by weight of *particulate stabiliser* (SP), $\phi_{SP}$, formed *in situ* or added as adjuvant, expressed with respect to the dispersed phase B:

$$\phi_{SP} = \frac{m_{SP}}{m_{SP} + m_B}$$

is between 0.001 and 0.5,

with $m_{SP}$ = mass of *particulate stabiliser*
$m_B$ = mass of phase B.

26. Emulsion according to Claim 25, **characterised in that** the said elastomer, the said solvent $\underline{a}$, the said solvent $\underline{b}$ and the said particulate stabiliser are as defined in Claims 3 to 12, 17, 18, 23 and 24.

27. Method of preparing a stable emulsion according to any one of Claims 1 to 24, **characterised in that** it comprises, in the case where the *particulate stabiliser* is added <u>before</u> the formation of the emulsion:

- the preparation of a phase A by dissolving the elastomer in an organic solvent $\underline{a}$ (phase A: elastomer solution in the organic solvent $\underline{a}$),

- the preparation of a phase B, by mixing an active chemical substance $\underline{x}$ in an organic solvent $\underline{b}$ which is not miscible with phase A (phase B: solution or dispersion of active substance in an organic solvent $\underline{b}$ not miscible with phase A),

- the addition to phase A or phase B, in the proportions as defined above, of a block or grafted copolymer serving essentially as a *dispersing copolymer* and a *particulate stabiliser* which is organophilic or made organophilic,

- the dispersion of phase B in phase A in order to obtain an emulsion in which phase A constitutes the continuous phase and phase B the dispersed phase.

28. Method of preparing a stable emulsion according to Claim 25 or Claim 26, **characterised in that** it comprises, in the case where the *particulate stabiliser* is added <u>before</u> the formation of the emulsion:

- the preparation of a phase A by dissolving the elastomer in an organic solvent $\underline{a}$ (phase A: elastomer solution in the organic solvent $\underline{a}$),

- the preparation of a phase B by mixing an active chemical substance $\underline{x}$ as defined in Claim 25 in an organic solvent $\underline{b}$ which is not miscible with phase A (phase B: solution or dispersion of active substance in an organic solvent $\underline{b}$ not miscible with phase A),

- the addition to phase A or phase B, in the proportions as defined above, or a *particulate stabiliser* which is organophilic or made organophilic, preferably formed from a block or grafted copolymer,

- the dispersion of phase B in phase A in order to obtain an emulsion in which phase A constitutes the continuous phase and phase B the dispersed phase.

29. Method of preparing a stable emulsion according to any one of Claims 1 to 24, **characterised in that** it comprises, in the case where the *particulate stabiliser* is added <u>during</u> or <u>after</u> the formation of the emulsion:

- the preparation of a phase A by dissolving the elastomer in an organic solvent $\underline{a}$ (phase A: elastomer solution in the organic solvent $\underline{a}$),

- the preparation of a phase B, by mixing an active chemical substance x in an organic solvent b which is not miscible with phase A (phase B: solution or dispersion of active substance in an organic solvent b not miscible with phase A),

- the addition to phase A or phase B, in the proportions as defined above, of a block or grafted copolymer serving essentially as a *dispersing copolymer*,

- the dispersion of phase B in phase A for obtaining an emulsion in which phase A constitutes the continuous phase and phase B the dispersed phase, and the addition, simultaneous or not, under agitation, of the *particulate stabiliser* which is organophilic or made organophilic.

30. Method of preparing a stable emulsion according to Claim 25 or Claim 26, **characterised in that** it comprises, in the case where the *particulate stabiliser* is added during or after the formation of the emulsion:

- the preparation of a phase A by dissolving the elastomer in an organic solvent a (phase A: elastomer solution in the organic solvent a),

- the preparation of a phase B by mixing an active chemical substance x as defined in Claim 25 in an organic solvent b which is not miscible with phase A (phase B: solution or dispersion of active substance in an organic solvent b not miscible with phase A),

- the dispersion of phase B in phase A in order to obtain an emulsion in which phase A constitutes the continuous phase and phase B the dispersed phase, and the addition, simultaneous or not, under agitation, of the *particulate stabiliser* which is organophilic or made organophilic, preferably formed from a block or grafted copolymer.

31. Elastomer film, **characterised in that** it is obtained by evaporating solvent a from an emulsion according to any one of Claims 1 to 26.

32. Method of preparing the elastomer film according to Claim 31, **characterised in that** it comprises:

(a) the preparation of an emulsion according to any one of Claims 1 to 26, and

(b) the evaporation of the organic solvent a, in order to obtain a film of elastomer containing, in the form of a stable dispersion, droplets of solvent b containing active chemical substance.

33. Application of the elastomer film according to Claim 31 as a coating for supports.

34. Glove, **characterised in that** it comprises an elastomer film according to Claim 31.

35. Fingerstall, **characterised in that** it comprises an elastomer film according to Claim 31.

36. Condom, **characterised in that** it comprises an elastomer film according to Claim 31.

37. Dressing, **characterised in that** it comprises an elastomer film according to Claim 31.

**Patentansprüche**

1. Stabile Emulsion von wenigstens einer chemischen Substanz x in einer Elastomerenlösung, die zur Verwendung zur Herstellung eines Elastomerenfilms geeignet ist, umfassend (1) eine Phase A, die ein in einem apolaren oder wenig polaren organischen Lösungsmittel a gelöstes Elastomer umfaßt, in der dispergiert ist (2) eine Phase B, die wenigstens die genannte chemische Substanz x in Lösung oder in einem polaren Lösungsmittel b dispergiert umfaßt, wobei die Phase B nicht mit der Phase A mischbar ist, und (3) wenigstens ein Dispergiermittel, ausgewählt aus der Gruppe bestehend aus Block- oder Pfropfcopolymeren, wobei die Emulsion **dadurch gekennzeichnet ist,**

• **daß** die Tröpfchen der dispergierten Phase B einen Durchmesser von $\geq 10$ µm besitzen,
• **daß** die genannte Emulsion zur Stabilisierung der genannten Phase B neben wenigstens einem *dispergierten*

*Copolymer* Sequenzen PolyA aufweist, die mit der Phase A kompatibel sind, und Sequenzen PolyB, die mit der Phase B kompatibel sind, wenigstens einen *partikulären Stabilisator*, ausgewählt aus der Gruppe, die gebildet ist aus festen organischen Verbindungen von einer Größe im Bereich zwischen 30 nm und 10 $\mu$m oder festen mineralischen Verbindungen von einer Größe im Bereich zwischen 5 nm und 10 $\mu$m, deren Oberflächenzustand organophil ist,

- **daß** die spezifische Fraktion $\phi_B$ der dispergierten Phase (Phase B) in der Emulsion ausgedrückt durch:

$$\phi_B = \frac{m_B}{m_B + m_A + m_{Elastomer}}$$

im Bereich zwischen 0,01 und 0,2 enthalten ist,

mit $m_B$ = Masse der Phase B (Lösungsmittel $\underline{b}$ + aktive chemische Substanz $\underline{x}$)
$m_A$ = Masse des Lösungsmittels $\underline{a}$
$m_{Elastomer}$= Masse des in $\underline{a}$ gelösten Elastomers;

- **daß** die spezifische Fraktion des Block- oder Pfropfcopolymers, $\phi_{CD}$, auf die dispergierte Phase B bezogen ausgedrückt als:

$$\phi_{CD} = \frac{m_{CD}}{m_{CD} + m_B}$$

im Bereich zwischen 0,001 und 0,3 enthalten ist, bevorzugt im Bereich zwischen 0,01 und 0,2,

mit $m_B$ = Masse der Phase B
$m_{CD}$ = Masse des dispergierten Copolymers; und

- **daß** die spezifische Fraktion des *partikulären Stabilisators* (SP), $\phi_{SP}$, der *in situ* gebildet oder als Adjuvans zugegeben wurde, bezogen auf die Phase B ausgedrückt als:

$$\phi_{SP} = \frac{m_{SP}}{m_{SP} + m_B}$$

im Bereich zwischen 0,001 und 0,5 enthalten ist,

mit $m_{SP}$ = Masse des *partikulären Stabilisators*
$m_B$ = Masse der Phase B.

2. Emulsion nach Anspruch 1, **dadurch gekennzeichnet, daß** die genannte spezifische Fraktion der Block- oder Pfropfcopolymeren, $\phi_{CD}$, im Bereich zwischen 0,001 und 0,2 enthalten ist, bevorzugt im Bereich zwischen 0,01 und 0,1.

3. Emulsion nach Anspruch 1 oder Anspruch 2, **dadurch gekennzeichnet, daß** wenn der *partikuläre Stabilisator* von organischer Natur ist, er aus der Gruppe ausgewählt ist, die gebildet ist aus organophilen Block- oder Pfropfcopolymeren , die identisch oder verschieden von denen der als dispergierte Copolymere verwendeten sind, wobei dieselben zur Bildung von Strukturen mit einem Durchmesser größer 30 nm geeignet sind, und Cellulosederivaten, wie Mikrocellulose, Stärke oder bestimmte, fein verteilte Polymere, die an ihrer Oberfläche ein Oberflächenbehandlungsmittel tragen, das geeignet ist den genannten *partikulären Stabilisator* organophil zu machen, wobei die spezifische Fraktion des Oberflächenbehandlungsmittels, das zum organophil machen der Oberfläche der Partikel, $\phi_{ATS}$, verwendet wurde, bezogen auf den *partikulären Stabilisator* ausgedrückt als:

$$\phi_{ATS} = \frac{m_{ATS}}{m_{SP} + m_{ATS}}$$

im Bereich zwischen 0,001 und 0,5 enthalten ist,

mit $m_{ATS}$ = Masse des Oberflächenbehandlungsmittels
$m_{SP}$ = Masse des *partikulären Stabilisators.*

4. Emulsion nach Anspruch 3, **dadurch gekennzeichnet, daß** die genannte spezifische Fraktion des Oberflächen-behandlungsmittels, $\phi_{ATS}$, im Bereich zwischen 0,001 und 0,1 enthalten ist.

5. Emulsion nach Anspruch 3 oder Anspruch 4, **dadurch gekennzeichnet, daß** die genannten organophilen Block-oder Pfropfcopolymere ausgewählt sind aus der Gruppe, gebildet von Copolymeren, die eine kristallisierbare Sequenz PolyB enthalten, wie z. B. Polyoxyethylen, Poly(ethylen), Polyamid, oder Polyester, wie Poly(caprolacton), und in der Phase A der Emulsion lösliche Sequenzen PolyA.

6. Emulsion nach Anspruch 1 oder Anspruch 2, **dadurch gekennzeichnet, daß** der *partikuläre Stabilisator* vom mineralischen Typ ist, wobei letzterer ausgewählt ist aus der Gruppe, umfassend Tone, Kieselsäuren, Talk, Kaolin oder Derivate dieser Produkte und an ihrer Oberfläche ein Oberflächenbehandlungsmittel tragen, das geeignet ist, den *partikulären Stabilisator* organophil zu machen, wobei die spezifische Fraktion des Oberflächenbehandlungsmittels, das zum organophil machen der Oberfläche der Partikel verwendet wurde, $\phi_{ATS}$, bezogen auf den *partikulären Stabilisator* ausgedrückt als:

$$\phi_{ATS} = \frac{m_{ATS}}{m_{SP} + m_{ATS}}$$

im Bereich zwischen 0,001 und 0,5 enthalten ist,

mit $m_{ATS}$ = Masse des Oberflächenbehandlungsmittels
$m_{SP}$ = Masse des *partikulären Stabilisators.*

7. Emulsion nach Anspruch 6, **dadurch gekennzeichnet, daß** die genannte spezifische Fraktion des Oberflächen-behandlungsmittels, $\phi_{ATS}$, im Bereich zwischen 0,001 und 0,1 enthalten ist.

8. Emulsion nach einem der Ansprüche 3 bis 7, **dadurch gekennzeichnet, daß** das Oberflächenbehandlungsmittel ausgewählt ist aus der Gruppe gebildet aus Blockoder Pfropfcopolymeren, die wenigstens eine in dem Lösungsmittel a lösliche Sequenz PolyA' und eine zum Absorbieren oder Verankern auf der Oberfläche des *partikulären Stabilisators* fähige Sequenz PolyB' enthalten, wobei die Sequenzen PolyA' und Poly B' identische oder unterschiedliche Sequenzen PolyA und PolyB und funktionalisierte Olygomere des Typs PolyA'-F sein können, in denen F eine chemische Funktion ist, die zur Absorption auf dem *partikulären Stabilisator* fähig ist.

9. Emulsion nach Anspruch 8, **dadurch gekennzeichnet, daß** die Sequenzen PolyA' ausgewählt sind aus den in dem apolaren oder wenig polaren Lösungsmittel a löslichen Polymeren, wie z. B. in der Gruppe, die Polydiene, Polyolefine, Polyether oder Silikone umfaßt, wie Polyisopren, Polybutadien, Polyisobuten, hydriertes Polybutadien oder hydriertes Polyisopren, Poly(4-tert.-butylstyrol), Polyoxypropylen, Polyoxybutylen, Polydimethylsiloxan, 2-Ethylhexylpoly(meth)acrylat, Laurylpoly(meth)acrylat, mischbar mit einer Elastomerenlösung in einem Lösungsmittel a, und wobei die Sequenzen PolyB' ausgewählt sind aus den Polymeren, die zur Absorption auf der Oberfläche des *partikulären Stabilisators* fähig sind, und ausgewählt sind aus der Gruppe, die Polyoxyethylen, Polyvinylpyrrolidon, Acrylpolyacide, Polyvinylalkohol und quatemisiertes oder nicht quaternisiertes Polyvinylypyridin umfaßt.

10. Emulsion nach Anspruch 9, **dadurch gekennzeichnet, daß** die Anteile der Sequenz PolyA', ausgedrückt als Masse in Bezug auf die Summe der Sequenzen PolyA'+PolyB', in einer Menge von 10 bis 90 % enthalten sind, bevorzugt in einer Menge von 20 bis 80 %, und die Anteile der Sequenzen PolyB' in einer Menge von 90 bis 10 % enthalten sind.

11. Emulsion nach Anspruch 9 oder Anspruch 10, **dadurch gekennzeichnet, daß** die Molmassen der Sequenzen PolyA' und PolyB' in einem Bereich zwischen 150 und 200 000 Dalton enthalten sind.

12. Emulsion nach Anspruch 8, **dadurch gekennzeichnet, daß** F ausgewählt ist aus der Gruppe gebildet aus Säurefunktion, Arminfunktionen oder Alkoholfunktion und die Gruppierung zur chemischen Reaktion mit der Oberfläche des *partikulären Stabilisators* geeignet ist, wie z. B. mit Epoxygruppen, Isocyanatgruppen oder Aziridingruppen.

13. Emulsion nach einem der Ansprüche 1 bis 12, **dadurch gekennzeichnet, daß** das dispergierte Blockcopolymer ausgebildet ist aus Diblockcopolymeren des Typs PolyA-Block-PolyB, Triblockcopolymeren des Typs PolyB-Block-PolyA-Block-PolyB (BAB), des Typs PolyA-Block-PolyB-Block-PolyA (ABA), des Typs PolyA-Block-PolyB-Block-PolyC (ABC), oder PolyA-Block-PolyC-Block-PolyB (ACB), oder allgemeiner aus Multiblockverbindungen, die die Sequenzen PolyA, PolyB und PolyC enthalten und das dispergierte Pfropfcopolymer ausgewählt ist aus den Verbindungen des Typs PolyA-Pfropf-PolyB, PolyB-Pfropf-PolyA, des Typs PolyA-Propf-PolyB und PolyC oder des Typs PolyC-Pfropf-PolyA und PolyB.

14. Emulsion nach Anspruch 13, **dadurch gekennzeichnet, daß** die Sequenz PolyA, ausgedrückt als auf die Summe der Sequenzen PolyA+PolyB bezogene Masse, in einer Menge zwischen 10 und 90 % enthalten sind, und die Anteile der Sequenzen PolyB in einer Menge zwischen 90 und 10 %, und die spezifischen Anteile der Sequenzen PolyC in einer Menge zwischen 0 und 50 % bezogen auf die Gesamtheit der Sequenzen enthalten sind.

15. Emulsion nach Anspruch 13 oder Anspruch 14, **dadurch gekennzeichnet, daß** die Molmassen der Sequenzen PolyA, PolyB und PolyC in dem Bereich zwischen 1000 und 500 000 Dalton enthalten sind.

16. Emulsion nach einem der Ansprüche 13 bis 15, **dadurch gekennzeichnet, daß** die Sequenzen PolyA, kompatibel mit dem Lösungsmittel $\underline{a}$, ausgewählt sind aus der Gruppe, die gebildet ist aus Polydienen, Polyolefinen, Polyehtem oder Silikonen, wie Polyisopren, Polybutadien, Polyisobuten, hydriertes Polybutadien oder hydriertes Polyisopren, Poly(4-tert.-butylstyrol), Polyoxypropylen, Polyoxybutylen, Polydimethylsiloxan, 2-Ethylhexylpoly(meth)acrylat, Laurylpoly(meth)acrylat, welches mischbar mit einer Elastomerenlösung in einem Lösungsmittel $\underline{a}$ ist, die Sequenzen PolyB, kompatibel mit dem Lösungsmittel $\underline{b}$, sind ausgewählt aus der Gruppe gebildet aus Polyoxyethylen, Polyvinylpyrrolidon, Acrylpolyaciden, Vinylpolyalkohol und quaternisiertem Polyvinylpyridin, und die Sequenzen PolyC, sei es kompatibel mit dem Lösungsmittel $\underline{a}$, sei es kompatibel mit dem Lösungsmittel $\underline{b}$, oder sei es nicht kompatibel mit den Lösungsmittteln $\underline{a}$ und $\underline{b}$, sind ausgewählt aus der Gruppe gebildet aus dem Acrylpolymeren oder Vinylpolymeren, wie Methylpolymethacrylat oder Polystyrol.

17. Emulsion nach einem der Ansprüche 1 bis 16, **dadurch gekennzeichnet, daß** das Elastomer ausgewählt aus der Gruppe gebildet von Polybutadien, Polyisopren, Polychloropren, SBR Copolymeren (*Styrolbutadienkautschuk*), NBR (*Nitrilbutadienkautschuk*), SBS (*Styrolbutadienstyrol*), SIS (*Styrolisoprenstyrol*), SEBS (*Styrolethylencobutylenstyrol*) alleine oder in Mischung mit einem oder mehreren Weichmachern oder Plastifizierungsmitteln.

18. Emulsion nach einem der Ansprüche 1 bis 16, **dadurch gekennzeichnet, daß** das Lösungsmitteln $\underline{a}$ insbesondere ausgewählt ist aus aliphatischen, aromatischen und alicyklischen Kohlenwasserstoffen, z. B. Methylcyclohexan, Toluol, Heptan oder einer Mischung derselben.

19. Emulsion nach einem der Ansprüche 1 bis 16, **dadurch gekennzeichnet, daß** die chemisch aktive Substanz $\underline{x}$, ausgewählt aus den Verbindungen, die fähig sind eine quasi sofortige Denaturierung von Proteinen durch einfachen Kontakt hervorzurufen, sei es durch chemische Reaktion, sei es durch einen physiko-chemischen Effekt, wie einer Veränderung der Oberflächenspannung.

20. Emulsion nach Anspruch 19, **dadurch gekennzeichnet, daß** die genannte chemisch aktive Substanz $\underline{x}$ ausgewählt ist aus den Bioziden.

21. Emulsion nach Anspruch 19 oder Anspruch 20, **dadurch gekennzeichnet, daß** das genannte Biozid ausgewählt ist aus quatemären Ammoniumverbindungen, bevorzugt aus Dimethyldidecylammoniumchlorid, Biguaniden, Phthalaldehyd, Phenoloder Benzolderivaten, Formalin, nichtionischen Tensiden, die wenigstens eine Polyoxyethylensequenz tragen, Hexamidin, Jodverbindungen von Polyvinylpyrrolidon, nicht-ionischen Tensiden mit virucider Aktivität, Bichromaten und Hypochloridverbindungen von Natrium und Kalium, die allein oder in Mischung verwendet werden.

22. Emulsion nach einem der Ansprüche 1 bis 21, **dadurch gekennzeichnet, daß** die spezifische Fraktion der chemisch aktiven Substanz $\underline{x}$, sei es $\phi_x$, ausgedrückt wird durch

$$\phi_x = \frac{m_x}{m_x + m_b} = \frac{m_x}{m_B}$$

im Bereich zwischen 0,01 und 0,7 enthalten ist, bevorzugt zwischen 0,1 und 0,4,

mit $m_x$ = Masse der chemischen aktiven Substanz x
$m_b$ = Masse des Lösungsmittels b
$m_B$ = Masse der dispergierten Phase B.

23. Emulsion nach einem der Ansprüche 1 bis 22, **dadurch gekennzeichnet, daß** das Lösungsmittel b nicht mit dem Lösungsmittel a mischbar ist und ausgewählt ist aus Polyolen, und bevorzugt aus Glyzerin, Ethylenglycol und den bei Raumtemperatur flüssigen Polyethylenglycolen, wobei deren Molmasse enthalten ist in dem Bereich zwischen 62 (Ethylenglycol) und 750 Dalton (PEG 750), und sämtliche anderen Verbindungen nicht mischbar mit dem Lösungsmittel a sind, wie z. B. Wasser, Dimethylsulfoxyd, Formamid oder Ethanolamin oder Mischungen dieser Lösungsmittel.

24. Emulsion nach einem der Ansprüche 1 bis 23, **dadurch gekennzeichnet, daß** sie mehrere *partikuläre Stabilisatoren* enthält.

25. Stabile Emulsion von wenigstens einer chemischen Substanz x in einer Elastomerenlösung, die zur Verwendung für die Herstellung eines elastomeren Films geeignet ist, umfassend (1) eine Phase A, die ein in einem apolaren oder wenig polaren organischen Lösungsmittel a gelöstes Elastomer umfaßt, in der dispergiert ist (2) eine Phase B, die wenigstens die genannte chemische Substanz x, in Lösung oder dispergiert in einem polaren, nicht mit der Phase A mischbaren Lösungsmittel b umfaßt, wobei die Emulsion **dadurch gekennzeichnet ist,**

- **daß** die Tröpfchen der dispergierten Phase B einen Durchmesser von ≥ 10 μm besitzen,
- **daß** die genannte Emulsion zur Stabilisierung der genannten dispergierten Phase B umfaßt (1) wenigstens eine chemische Substanz x, wobei letztere tensioative Eigenschaften besitzt und die Rolle eines Dispersionsmittels spielt, wobei die genannte chemische Substanz x ausgewählt ist aus der Gruppe gebildet aus quaternären Ammoniumverbindungen, nicht-ionischen Tensiden, die wenigstens eine Polyoxyethylensequenz tragen, und nicht-ionischen Tensiden mit virucider Aktivität, und (2) wenigstens einen *partikulären Stabilisator,* wie er in Anspruch 1 definiert ist, bevorzugt gebildet ausgehend von einem Block- oder Pfropfcopolymer,
- **daß** die spezifische Fraktion $\phi_B$ der dispergierten Phase (Phase B) in der Emulsion ausgedrückt durch:

$$\phi_B = \frac{m_B}{m_B + m_A + m_{Elastomer}}$$

in dem Bereich zwischen 0,01 und 0,2 enthalten ist,

mit $m_B$ = Masse der Phase B (Lösungsmittel b + aktive chemische Substanz x)
$m_A$ = Masse des Lösungsmittels a
$m_{Elastomer}$ = Masse des in a gelösten Elastomers;

- **daß** die spezifische Fraktion der chemisch aktiven Substanz x, sei es $\phi_x$, ausgedrückt durch

$$\phi_x = \frac{m_x}{m_x + m_b} = \frac{m_x}{m_B}$$

und in dem Bereich zwischen 0,01 und 0,7 enthalten ist, bevorzugt zwischen 0,1 und 0,4,

mit $m_x$ = Masse der chemischen aktiven Substanz x
$m_b$ = Masse des Lösungsmittels b
$m_B$ = Masse der dispergierten Phase B,

- **daß** die spezifische Fraktion des *partikulären Stabilisators* (SP), $\phi_{SP}$, gebildet *in situ* oder als Adjuvans zugegeben, bezogen auf die dispergierte Phase B ausgedrückt als

$$\phi_{SP} = \frac{m_{SP}}{m_{SP} + m_B}$$

und enthalten ist in dem Bereich zwischen 0,001 und 0,5,

mit $m_{SP}$ = Masse des *partikulären Stabilisators*
$m_B$ = Masse der Phase B.

26. Emulsion nach Anspruch 25, **dadurch gekennzeichnet, daß** das genannte Elastomer, das genannte Lösungsmittel a, das genannte Lösungsmittel b und der genannte Stabilisator solche sind, wie in den Ansprüchen 3 bis 12, 17, 18, 23 und 24 definiert.

27. Verfahren zur Herstellung einer stabilen Emulsion gemäß einem der Ansprüche 1 bis 24, **dadurch gekennzeichnet, daß** es in dem Fall, in dem der *partikuläre Stabilisator* vor der Bildung der Emulsion zugegeben wird, die Schrittte umfaßt:

  - Herstellung einer Phase A durch Lösen des Elastomeren in einem organischen Lösungsmittel a (Phase A: Elastomerenlösung in dem organischen Lösungsmittel a),
  - Herstellung einer Phase B, durch Mischen einer chemisch aktiven Substanz x in einem nicht mit der Phase A mischbaren organischen Lösungsmittel b (Phase B: Lösung oder Dispersion der aktiven Substanz in einem nicht mit der Phase A mischbaren organischen Lösungsmittel b),
  - Zugabe eines Block- oder Pfropfcopolymers zu der Phase A oder zu der Phase B in den oben definierten Anteilen, wobei dieselben im wesentlichen als *dispergiertes Copolymer* oder organophiler oder organophil gemachter *partikulärer Stabilisator* dienen,
  - Dispergieren der Phase B in der Phase A zum Erhalt einer Emulsion, in welcher die Phase A die kontinuierliche Phase bildet und die Phase B die dispergierte Phase.

28. Verfahren zur Herstellung einer stabilen Emulsion gemäß Anspruch 25 oder Anspruch 26, **dadurch gekennzeichnet, daß** es in dem Fall, in dem der *partikuläre Stabilisator* vor der Bildung der Emulsion zugegeben wird, die Schritte umfaßt:

  - Herstellung einer Phase A durch Auflösen des Elastomeren in einem organischen Lösungsmittel a (Phase A: Elastomerenlösung in dem organischen Lösungsmittel a),
  - Herstellung einer Phase B durch Mischen einer chemisch aktiven Substanz x, wie sie in Anspruch 25 definiert ist, in einem nicht mit der Phase A mischbaren organischen Lösungsmittel b (Phase B: Lösung oder Dispersion der aktiven Substanz in einem nicht mit der Phase A mischbaren organischen Lösungsmittel b),
  - Zugabe zur Phase A oder zur Phase B, in den oben definierten Anteilen, eines organophilen oder organophil gemachten *partikulären Stabilisators,* bevorzugt gebildet ausgehend von einem Block- oder Pfropfcopolymer,
  - Dispergieren der Phase B in der Phase A zum Erhalt einer Emulsion, in welcher die Phase A die kontinuierliche Phase bildet und die Phase B die dispergierte Phase.

29. Verfahren zur Herstellung einer stabilen Emulsion gemäß einem der Ansprüche 1 bis 24, **dadurch gekennzeichnet, daß** es in dem Fall, in dem der *partikuläre Stabilisator* während oder nach der Bildung der Emulsion zugegeben wird, die Schritte umfaßt:

  - Herstellung einer Phase A durch Auflösen des Elastomeren in einem organischen Lösungsmittel a (Phase A: Elastomerenlösung in dem organischen Lösungsmittel a),
  - Herstellung einer Phase B durch Mischen einer chemisch aktiven Substanz x in einem nicht mit der Phase A mischbaren organischen Lösungsmittel b (Phase B: Lösung oder Dispersion der aktiven Substanz in einem nicht mit der Phase A mischbaren organischen Lösungsmittel b),
  - Zugabe eines Block- oder Pfropfcopolymers, das im wesentlichen als *dispergiertes Copolymer* dient, zur Phase A oder zur Phase B in den oben definierten Anteilen,
  - Dispergieren der Phase B in der Phase A zum Erhalt einer Emulsion, in welcher die Phase A die kontinuierliche Phase bildet und die Phase B die dispergierte Phase und gleichzeitige oder nicht gleichzeitige Zugabe, unter Rühren, des organophilen oder organophil gemachtem *partikulärem Stabilisators.*

**30.** Verfahren zur Herstellung einer stabilen Emulsion nach Anspruch 25 oder Anspruch 26, **dadurch gekennzeichnet, daß** es in dem Fall, in dem der *partikuläre Stabilisator* während oder nach der Bildung der Emulsion zugegeben wird, die Schritte umfaßt:

- Herstellung einer Phase A durch Auflösen des Elastomeren in einem organischen Lösungsmittel a (Phase A: Elastomerenlösung in dem organischen Lösungsmittel a),
- Herstellung einer Phase B durch Mischen einer chemisch aktiven Substanz x, wie sie in Anspruch 25 definiert ist, in einem nicht mit der Phase A mischbaren organischen Lösungsmittel b (Phase B: Lösung oder Dispersion der aktiven Substanz in einem nicht mit der Phase A mischbaren organischen Lösungsmittel b),
- Dispergieren der Phase B in der Phase A zum Erhalt einer Emulsion, in welcher die Phase A die kontinuierliche Phase darstellt und die Phase B die dispergierte Phase, und gleichzeitige oder nicht gleichzeitige Zugabe, unter Rühren, des organophilen oder organophil gemachten *partikulären Stabilisators,* bevorzugt gebildet ausgehend aus einem Block- oder Pfropfcopolymer.

**31.** Elastomerenfilm, **dadurch gekennzeichnet, daß** er durch Verdampfen des Lösungsmittels a aus einer Emulsion nach den Ansprüchen 1 bis 26 erhalten wird.

**32.** Verfahren zur Herstellung eines Elastomerenfilms gemäß Anspruch 31, **dadurch gekennzeichnet, daß** es umfaßt:

(a) Herstellung einer Emulsion gemäß der Ansprüche 1 bis 26 und
(b) Verdampfen des organischen Lösungsmittels a zum Erhalt eines Elastomerenfilms, welcher in Form einer stabilen Dispersion Tröpfchen des Lösungsmittels b enthält, die mit chemisch aktiver Substanz beladen sind.

**33.** Verwendung des Elastomerenfilms nach Anspruch 31 als Trägermaterial.

**34.** Handschuh, **dadurch gekennzeichnet, daß** er einen Elastomerenfilm nach Anspruch 31 aufweist.

**35.** Fingersatz, **dadurch gekennzeichnet, daß** er einen Elastomerenfilm nach Anspruch 31 aufweist.

**36.** Präservativ, **dadurch gekennzeichnet, daß** es einen Elastomerenfilm nach Anspruch 31 aufweist.

**37.** Pflaster, **dadurch gekennzeichnet, daß** es einen Elastomerenfilm gemäß Anspruch 31 aufweist.

FIGURE 1

FIGURE 2

FIGURE 3

composé ORGANIQUE

composé MINERAL

en tant
qu'adjuvant

structure formée in situ par un copolymère
de même nature que celui utilisé
comme agent dispersant

en tant
qu'adjuvant

- microcellulose
- amidon
- microlatex...
- structure formée par un copolymère
de nature chimique différente de celui utilisé
comme agent dispersant

- silice
- bentone
- talc
- kaolin...

SP rendu "organophile"
par un agent de traitement
de surface

produit commercial
déjà traité

SP rendu "organophile"
par un agent de traitement
de surface

FIGURE 4

phase A

phase B

- solution d'élastomère dans
un solvant a

- solvant b et substance
chimique active x

ajout du copolymère
dispersant

OU

ajout du copolymère
dispersant

AJOUT PHASE B (possibilité 1) PUIS EMULSION

alternative 1

stabilisant particulaire
sous forme d'adjuvant

stabilisant particulaire : structure associée
apportée directement par le copolymère
dispersant

structure associée
formée par un copolymère
différent de celui utilisé
pour la dispersion

autre particule solide organique
ou minéral

AJOUT PHASE B (possibilité 2) PUIS EMULSION

alternative 2

FIGURE 5

36

FIGURE 6

FIGURE 7